# EUROPEAN PATENT SPECIFICATION

(11) **EP 4 608 493 B1**
(45) Date of publication and mention of the grant of the patent: **09.09.2026**
(21) Application number: 23786755.1
(22) Date of filing: 03.10.2023
(51) Int. Cl.: A61N 1/05, A61B 17/00

(54) **SYSTEM FOR LEAD ANCHORING AND WOUND CLOSURE**
SYSTEM ZUR VERANKERUNG VON LEITUNGEN UND ZUM WUNDVERSCHLUSS
SYSTÈME D'ANCRAGE DES SONDES ET DE FERMETURE DES PLAIES

(30) Priority: 28.10.2022 US 202263381410 P
(43) Date of publication of application: 03.09.2025
(73) Proprietor: Medtronic, Inc., Minneapolis, Minnesota 55432 (US)
(72) Inventor: HOFFMAN, Matthew J., Minneapolis, Minnesota 55432 (US); MARSHALL, Mark T., Minneapolis, Minnesota 55432 (US)
(74) Representative: Zimmermann & Partner Patentanwälte mbB
(86) International application number: PCT/IB2023/059921
(87) International publication number: WO 2024/089499

(56) References cited:
- US-A1- 2005 131 484
- US-A1- 2006 122 660
- US-A1- 2014 155 973
- US-A1- 2019 105 489
- US-B2- 11 096 757
- US-B2- 7 369 897

## Description

### TECHNICAL FIELD

This disclosure relates generally to implant tool systems.

### BACKGROUND

Some types of implantable medical devices (IMDs), such as cardiac pacemakers or implantable cardioverter defibrillators systems, may be used to provide cardiac sensing and therapy for a patient via one or more electrodes. Some IMDs include one or more implantable medical electrical leads that include one or more electrodes. The leads may be configured such that the electrodes may, as examples, be implanted within the heart (e.g., transvenous leads) or outside of the heart and vasculature (e.g., extravascular leads). Once the leads are implanted, tines or other fixation elements attached to various locations of the leads may be deployed to prevent the leads from shifting or moving.

Implantable medical leads may be adapted to treat a wide variety of cardiac dysfunctions. An implantable medical lead may be navigated through vasculature or extravascular space of a patient to reach one or more target locations for sensing and/or therapy delivery. An electrode supported by the implantable medical lead may establish electrical communication with tissues of the heart to sense cardiac signals generated by the heart and/or deliver cardiac pacing to the patient.

US 11,096,757 B2 relates to implantable medical lead indicators. US 2006/122660 A1 relates to a method and system for modulating sacral nerves and/or its branches in a patient to provide therapy for urological disorders and/or fecal incontinence, using rectangular and/or complex electrical pulses. US 2005/131484 A1 relates to a method and system for providing pulsed electrical stimulation to provide therapy for erectile/sexual dysfunction, prostatitis, prostatitis pain, and chronic pelvic pain.

### SUMMARY

In general, the disclosure describes systems, techniques, and devices for medical device anchoring systems in patient tissue. The present disclosure describes example lead anchoring systems that help a clinician or other user to anchor an implantable lead in patient tissue, as well as help the clinician close an incision wound for entry of the medical device into the patient tissue. Such lead anchoring systems may include an implantable medical lead and anchoring sleeve. The anchoring sleeve may include an engagement mechanism, wherein the anchoring sleeve and/or engagement mechanism may be configurable between an expanded state and a contracted state.

In some examples a medical device system includes an implantable medical lead configured to be delivered through an incision site on a body of a patient and an anchoring sleeve surrounding a portion of the lead and including an engagement mechanism configured to: engage with patient tissue at the incision site; and assist closure of an incision in the patient tissue at the incision site.

In some examples a method, which is not claimed as such but is useful for understanding the claimed subject-matter, includes: inserting an implantable medical lead of a medical device system through an incision site on a body of a patient; guiding an anchoring sleeve of the medical device system to the incision site, wherein the anchoring sleeve surrounds a portion of the lead; and engaging the anchoring sleeve with patient tissue at the incision site via an engagement mechanism of the anchoring sleeve, wherein the engagement mechanism is configured to assist closure of an incision in the patient tissue at the incision site.

In some examples an anchoring sleeve includes an engagement mechanism configurable between an expanded state and a contracted state, wherein: the engagement mechanism is configured to engage with patient tissue at an incision site in patient tissue, and the engagement mechanism is configured to assist closure of an incision in the patient tissue at the incision site when the engagement mechanism transitions from the expanded state to the contracted state.

The details of one or more examples are set forth in the accompanying drawings and the description below. Other features, objects, and advantages will be apparent from the description and drawings, and from the claims.

### BRIEF DESCRIPTION OF THE DRAWINGS

FIG. 1 is a conceptual drawing illustrating an example lead anchoring system in conjunction with a patient, in accordance with one or more techniques of the disclosure.
FIG. 2 is a conceptual drawing illustrating the example lead anchoring system of FIG. 1 at a different incision site in the body of a patient, in accordance with one or more techniques of the disclosure.
FIG. 3 is a conceptual drawing illustrating an example lead anchoring system at an incision site, in accordance with one or more techniques of the disclosure.
FIG. 4 is a conceptual drawing illustrating an example lead anchoring system including an introducer, in accordance with one or more techniques of the disclosure.
FIG. 5A is a conceptual drawing illustrating an example tine assembly in a constrained state, in accordance with one or more techniques of the disclosure.
FIG. 5B is a conceptual drawing illustrating the example time assembly of FIG. 5A in an unconstrained state, in accordance with one or more techniques of the disclosure.
FIG. 6 is a conceptual drawing illustrating an example anchoring sleeve including a coiling tine to engage with patient tissue, in accordance with one or more techniques of the disclosure.
FIG. 7A is a conceptual drawing illustrating an example lead anchoring system including an expanding feature in an expanded state, in accordance with one or more techniques of the disclosure.
FIG. 7B is a conceptual drawing illustrating the example lead anchoring system of FIG. 7A, including the expanding feature in a contracted state, in accordance with one or more techniques of the disclosure.
FIG. 7C is a conceptual drawing illustrating the example lead anchoring system of FIGS. 7A and 7B including one or more elastic spines, in accordance with one or more techniques of the disclosure.
FIG. 8A is a conceptual drawing illustrating an example strut assembly of an anchoring sleeve in an expanded state, in accordance with one or more techniques of the disclosure.
FIG. 8B is a conceptual drawing illustrating the example strut assembly of FIG. 8A in a contracted state, in accordance with one or more techniques of the disclosure.
FIG. 9 is a conceptual drawing illustrating an example tie mechanism for a strut assembly, in accordance with one or more techniques of the disclosure.
FIG. 10A is a conceptual drawing illustrating an example outer side of patient tissue at an incision site, in accordance with one or more techniques of the disclosure.
FIG. 10B is a conceptual drawing illustrating an example inner side of patient tissue at the incision site of FIG. 10A, in accordance with one or more techniques of the disclosure.
FIG. 11 is a flow diagram of an example technique for closing an incision in patient tissue using an anchoring sleeve configured to assist closure of the incision, in accordance with one or more techniques of the disclosure.

### DETAILED DESCRIPTION

When implanting a lead of an implantable medical device (IMD) within a patient, a clinician may be required to make incisions in patient tissues to create an access site for implantation of the lead. After implantation, the clinician may close the incision wounds. Closure of the incision wounds may be difficult, especially when the incision to be closed is deep within the patient's body (e.g., an incision in the mediastinum), and may require stable anchoring of the lead so as not to dislodge the lead during the closure. Existing methods for wound closure and lead securing use resorbable and non-resorbable sutures with surgical techniques that provide inconsistent results due to the difficulty of the operation for a physician.

This disclosure describes example lead anchoring systems that help a clinician or other user to anchor the lead in patient tissue, as well as help close the incision wound, thereby simplifying the procedure, and providing safer and more consistent results for wound closure. Such lead anchoring systems may include an implantable medical lead configured to be delivered through an incision site on the body of the patient. The lead anchoring systems may also include an anchoring sleeve surrounding a portion of the lead. The anchoring sleeve may include an engagement mechanism to attach the anchoring sleeve to patient tissue at the incision site. One or more of the anchoring sleeve and/or the engagement mechanism may be configurable between an expanded and a contracted state, wherein when the anchoring sleeve and/or the engagement mechanism transition from the expanded to the contracted state, the anchoring sleeve and/or engagement mechanism assist closure of the incision in the patient tissue.

The lead anchoring system may include an introducer or dilator configured to deliver the anchoring sleeve along the implantable medical lead to the incision site. The anchoring sleeve may be positioned around the introducer for delivery to the incision site. In examples where the anchoring sleeve is configurable between an expanded and a contracted state, the introducer may hold the anchoring sleeve in the expanded state, such that the anchoring sleeve applies a compressive force to the introducer. The compressive force may prevent air ingress into the patient's body during implantation. After the engagement mechanism of the anchoring sleeve has engaged the patient tissue, the introducer may be removed, allowing the anchoring sleeve to further contract onto the implanted lead. The engagement mechanism, being attached to the anchoring sleeve, may contract along with the anchoring sleeve, pulling patient tissue together as the anchoring sleeve compresses. The introducer may be made from PTFE or another low friction material configured to allow removal of the introducer from the anchoring sleeve.

In some examples, the engagement mechanism may include a plurality of tines, wherein the engagement mechanism is configured to deploy the tines into patient tissue. The engagement mechanism and tines may be made of shape-memory allows (e.g., nitinol), or may be mechanically actuated and fastened using clips or zip-tie structures. The plurality of tines may be configured to transition from a constrained state to an unconstrained state, wherein transitioning from the constrained state to the unconstrained state causes the plurality of tines to engage with the patient tissue.

In some examples, the engagement mechanism includes one or more tine assemblies. The tine assemblies may include one or more surface features configured to allow a tool to grip the tine assembly and a compression fitting that also includes one or more surface features configured to allow a tool to grip the compression fitting. The tine assemblies may also include two or more tines of the plurality of tines, wherein the two or more tines extend in the direction of a longitudinal axis of the tine assembly when the two or more tines are in the constrained state. The two or more tines may be configured to transition between the constrained state and the unconstrained state in response to the compression fitting sliding along the longitudinal axis of the tine assembly.

In some examples, the engagement mechanism includes a corkscrew fixation mechanism. The corkscrew fixation mechanism may include one or more tines configured to engage with patient tissue when the corkscrew mechanism is rotated around a longitudinal axis of the anchoring sleeve. Further rotation may cause the patient tissue to cinch together around the corkscrew fixation mechanism, drawing the patient tissue together.

In some examples, the anchoring sleeve and engagement mechanism may include an expanding feature configurable between an expanded and a contracted state. For example, the expanding feature may include a plurality of metal spines connected to one another by membrane or envelope. When compressed longitudinally, the plurality of metal spines may spiral radially away from the anchoring sleeve, expanding the membrane. When not subject to a longitudinal compressive force, the plurality of metal spines may be configured to spring back into the contracted state.

The anchoring sleeve and/or engagement mechanism may be configured to expand and contract by means of a strut assembly. For example, the strut assembly may include a plurality of struts defining an inner and outer diameter of the strut assembly. The plurality of struts may be connected to one another by a plurality of rotatable connections, such that when rotated, the strut assembly transitions between an expanded state and a contracted state. Tines on the struts that define the outer diameter of the strut assembly may engage patient tissue, and pull patient tissue together when the strut assembly is contracted. After implantation of the lead and anchoring sleeve, a zip tie or other fastener may be used to hold the strut assembly in the contracted state.

In this way, the techniques of this disclosure allow placement of the anchoring sleeve at the incision site and at least partial closure of the incision wound to be performed simultaneously with the same device. In some examples, the techniques of this disclosure may allow closure of the incision wound automatically by the anchoring sleeve after placement, without the need for delicate suture procedures. In some examples, the difficulty of additional suturing procedures may be made simpler because of the partial closure of the incision wound.

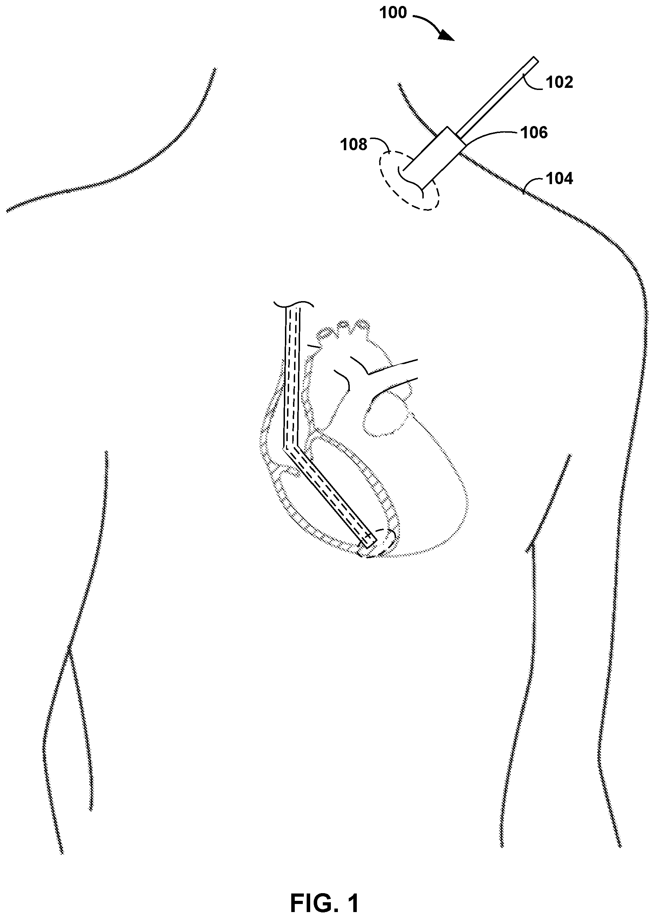

FIG. 1 is a conceptual drawing illustrating an example medical device system 100 (e.g., a lead anchoring system) in conjunction with a patient 104, in accordance with one or more techniques of the disclosure.

Medical device system 100 includes implantable medical lead 102 configured to be delivered through incision site 108 on a body of patient 104 and anchoring sleeve 106 surrounding a portion of lead 102. Anchoring sleeve 106 may include an engagement mechanism (not pictured) configured to engage with patient tissue at incision site 108 and assist closure of an incision in the patient tissue at incision site 108. In the example of FIG. 1, lead 102 is implanted at least partially within patient 104 through incision site 108, and lead 102 may be anchored in place by anchoring sleeve 106. Although FIG. 1 depicts lead 102 leading to an implant site in the heart of patient 104, components of system 100 described herein may be utilized with various types of implant tool systems, such as implant tool systems for delivering IMDs configured to deliver electrical therapy (e.g., cardiac electric therapy, neurostimulation), or other implant tool systems. Furthermore, although primarily described herein as being an implantable medical lead (e.g., a cardiac lead), lead 102 may be another type of device, such as a catheter. In addition, it should be noted that system 100 may not be limited to treatment of a human patient. System 100 may be implemented in non-human patients, such as primates, canines, equines, pigs, ovines, bovines, felines, etc. These non-human patients may undergo clinical or research therapies that may benefit from the subject matter of this disclosure.

Lead 102 may include a proximal end and a distal end. In some examples the distal end may include one or more electrodes and rest against or be attached to patient tissue within patient 104. In some examples the proximal end may be connected to a medical device, for example an implantable cardiac defibrillator (ICD). In some examples, the medical device is configured to be implanted in the body of patient 104. In some examples, the medical device may remain on the exterior of patient 104 with only lead 102 entering patient 104. Although only one lead is shown in FIG. 1, in some examples, medical device system 100 includes multiple leads 102, and multiple anchoring sleeves 106 for each of leads 102.

Although shown entering the body from an external source in FIG. 1, in some examples, lead 102 may be entirely implanted within patient 104. For example, lead 102 may be connected to an ICD that is subcutaneously implanted on the left midaxillary of patient 104, where lead 102 extends subcutaneously from the ICD to a treatment site within patient 104. In some examples, incision site 108 is not an incision in the skin, but in interior bodily tissue of patient 104, e.g., the mediastinal pleurae, the fascia covering the pectoralis major muscle, or whatever may form the pocket floor during an implant procedure. In some examples, incision site 108 includes incision through both skin tissue of patient 104 as well as other connective tissues of patient 104. Although shown in FIG. 1 as predominantly straight, in some examples, lead 102 and/or anchoring sleeve 106 may bend or turn within patient 104 at an incision site 108.

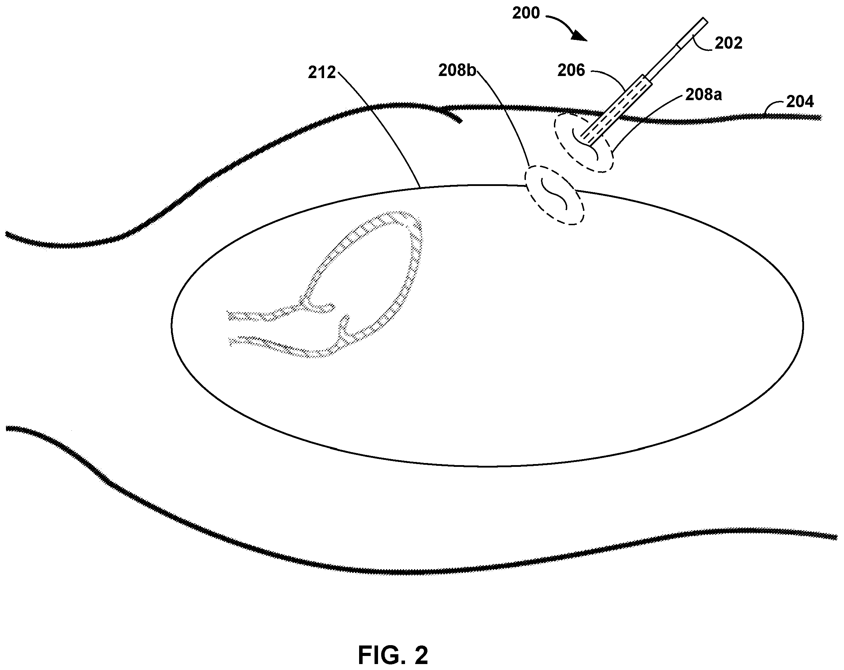

In some examples, a clinician may insert lead 102 into and through a patient's vasculature to a target site within a body of patient 104 (e.g., tissue of a heart of patient 104) where a medical procedure may be undertaken. In some examples, the clinician may insert lead 102 under patient 104's sternum and to a target site within the body of patient 104, rather than through the vasculature, as shown in FIG. 2. Similarly, although the incision site is pictured in FIG. 1 as being located in the upper chest, in other examples it may be located anywhere on the body of patient 104. Lead 102 may be temporary (e.g., lead 102 may be a temporary pacing balloon lead) or permanent. The length of lead 102 may vary.

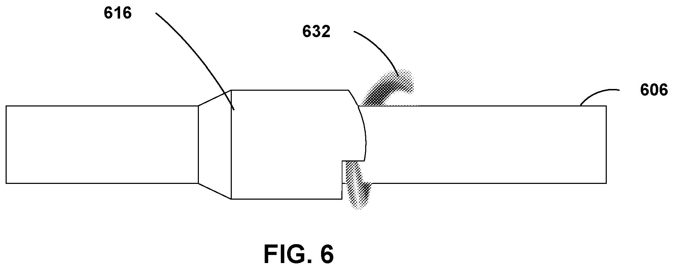

Anchoring sleeve 106 may prevent lead 102 from shifting or moving (e.g., in a proximal direction) once fixed to tissue at incision site 108. For example, anchoring sleeve 106 may attach or otherwise fixate to lead 102 such that, when anchoring sleeve 106 is fixed to tissue at incision site 108, the portion of lead 102 attached to anchoring sleeve 106 may not move with respect to the tissue. Anchoring sleeve 106 may include an engagement mechanism to engage with patient tissue. The engagement mechanism may include, for example, one or more elongated tines such as fixation tines configured to substantially maintain an orientation of lead 102 with respect to incision site 108. In some examples, the engagement mechanism may include adhesives or other mechanism for engaging with patient tissue. The engagement mechanism may include any shape, for example a helically-shaped fixation element, as shown in FIG. 6. In some examples, the engagement mechanism may be attached to anchoring sleeve 106, or otherwise manufactured as a part of anchoring sleeve 106. In some examples, the engagement mechanism may be retractable/advanceable/assembled onto separate anchoring sleeves before implantation of lead 102.

In some examples, anchoring sleeve 106 may assist closure of the incision in the patient tissue when a clinician or technician anchors anchoring sleeve 106 to patient tissue. For example, as a helical fixation element (discussed in more detail below in FIG. 6) turns about a longitudinal axis to engage with patient tissue, the helical fixation element may also pull patient tissue inward towards anchoring sleeve 106, at least partially closing the incision in patient tissue.

Anchoring sleeve 106 may be configurable between an expanded and a contracted state, by way of the engagement mechanism or separate from the functioning of the engagement mechanism. When anchoring sleeve 106 transitions from the expanded to the contracted state, anchoring sleeve 106 may assist closure of the incision in the patient tissue. In some examples, the anchoring sleeve includes one or more shape-memory tines held in a high energy state. When the shape-memory tines are allowed to transition to their low-energy state, they may engage with patient tissue and pull it inwards towards the anchoring sleeve, at least partially closing the incision. In some examples, the anchoring sleeve includes an expanding feature, where an outer surface of the expanding feature includes tines configured to engage with patient tissue. The expanding feature may engage with patient tissue at incision site 108 when anchoring sleeve 106 is in the expanded state, and may pull patient tissue together when transitioned to the contracted state.

By incorporating anchoring sleeve 106 into medical device system 100, wherein anchoring sleeve 106 assists closure of the incision, physicians may be saved the time and difficulty of performing attachment methods involving suturing other anchoring sleeves to patient tissue.

FIG. 2 is a conceptual drawing illustrating an example medical device system 200 (e.g., a lead anchoring system) at a different incision site in the body of a patient, in accordance with one or more techniques of the disclosure. In the example shown in FIG. 2, an incision has been made through the skin of patient 204 at a first incision site 208a, for example to form a pocket in patient 204 tissue for implantation of an IMD (not shown) connected to lead 202. A second incision site 208b may allow lead 202 to extend from the pocket and further into the body of patient 204. System 200 may be substantially similar to system 100 of FIG. 1. Similarly, lead 202 and anchoring sleeve 206 may be substantially similar to lead 102 and anchoring sleeve 106, respectively, shown in FIG. 1.

Lead 202 may include a proximal end that is connected to an IMD (e.g., an ICD) and a distal end that includes one or more electrodes. The incision at incision sites 208 may be made in order to implant the IMD and lead 202 in patient 204. For example, once implanted lead 202 may extend subcutaneously from the implanted IMD toward the xiphoid process. At a location near the xiphoid process lead 202 may bend or turn and extend superior upward in the substernal space. In one example, lead 202 may be placed in the mediastinum 212 and, more particularly, in the anterior mediastinum. The anterior mediastinum is bounded laterally by pleurae, posteriorly by the pericardium, and anteriorly by the sternum. In some examples, lead 202 may be implanted within the mediastinum such that one or more electrodes of the lead are located over a cardiac silhouette of the ventricle as observed via fluoroscopy. In some examples, lead 202 may be located substantially centered under the sternum. In other examples lead 202 may be implanted such that it is offset laterally from the center of the sternum. Although described herein as being implanted in the substernal space, the mediastinum, or the anterior mediastinum, lead 202 may be implanted in other extra-pericardial locations.

Medical device system 200 includes anchoring sleeve 206 to prevent lead 202 from shifting or moving (once anchoring sleeve 206 is fixed to tissue at incision site 208b) during the implantation procedure. Anchoring sleeve 206 may be positioned partially in the subcutaneous tissue and partially within the substernal space. Anchoring sleeve 206 may be coupled to patient 204 at one or more points along the length of anchoring sleeve 206.

In some existing procedures, a physician must couple an anchoring sleeve to a patient via one or more sutures that are sewn through patient tissue at an incision site and tied or otherwise wrapped around the anchoring sleeve. Significant force must be applied to the sutures to tighten them and hold the anchoring sleeve and patient tissue together. This procedure can be difficult, as the physician is attempting to manipulate the sutures in a moist environment through a small hole some depth within patient tissue (e.g., three inches).

In order to reduce the time and difficulty of the lead implant procedure, anchoring sleeve 206 may include features to engage with patient tissue and assist closure of the incision.

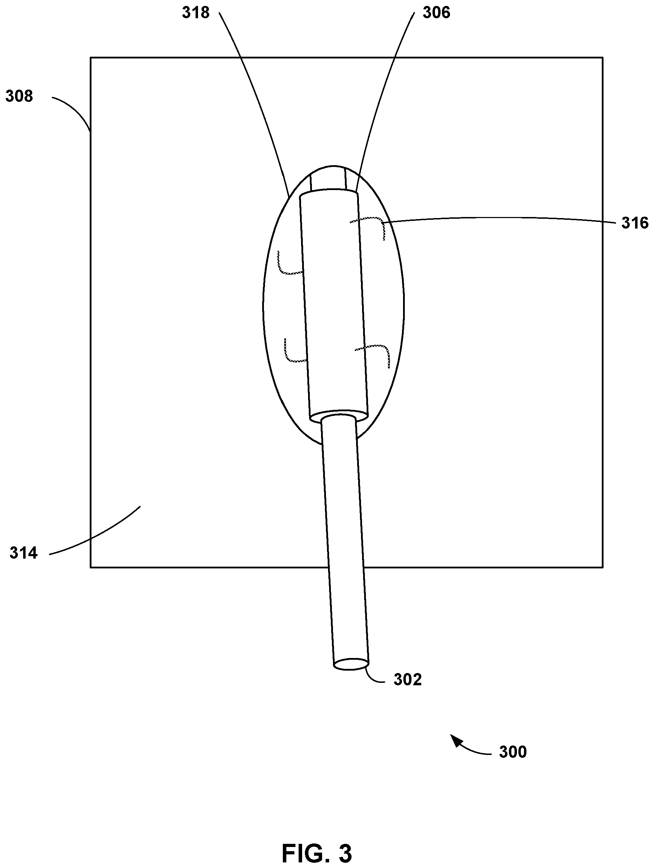

FIG. 3 is a conceptual drawing illustrating an example lead anchoring system 300 at an incision site 308, in accordance with one or more techniques of the disclosure. Lead anchoring system 300 may include lead 302 and anchoring sleeve 306. Lead 302 and anchoring sleeve 306 may be substantially similar to lead 102 and anchoring sleeve 106, respectively, shown in FIG. 1. Lead 302 may need to be implanted into patient tissue 314 at an incision site 308. Incision site 308 may be substantially similar to incision site 208b shown in FIG. 2 and/or incision site 108 shown in FIG. 1. Anchoring sleeve 306 may be configured to attach to lead 302 through any method known in the art.

Anchoring sleeve 306 may include an engagement mechanism 316 configured to engage with patient tissue 314 at incision site 308 and assist closure of incision 318 in patient tissue 314 at incision site 308. Engagement mechanism 316 may include one or more tines, an adhesive, or another method for attaching to patient tissue 314. Anchoring sleeve 306 and/or engagement mechanism 316 may be configurable between an expanded state and a contracted state, wherein transitioning from the expanded state to the contracted state assists closure of incision 318 in patient tissue 314 at incision site 308.

Engagement mechanism 316 may be configured to engage with patient tissue 314 at incision site 308 while anchoring sleeve is disposed within incision 318. Engagement mechanism 316 may engage with the severed portions of patient tissue 314 representing the inner walls defined by incision 318 while anchoring sleeve 306 is in the expanded state. When anchoring sleeve 306 transitions to the contracted state, engagement mechanism 316 may pull incision 318 at least partially closed by pulling the inner walls of incision 318 towards one another.

In some examples, anchoring sleeve 306 and/or engagement mechanism 316 may be made of shape memory alloys and or polymeric structures that can expand and be self-collapsing. Anchoring sleeve 306 and engagement mechanism 316 may be held in the expanded state while being navigated to incision site 308 and engaged with patient tissue 314. Once engagement mechanism 316 is attached to patient tissue 314, anchoring sleeve 306 and engagement mechanism 316 may be allowed to self-collapse to transition to the contracted state, pulling incision 318 at least partially closed. In some examples anchoring sleeve 306 and/or engagement mechanism 316 may be collapsed through a mechanically actuated force.

Although engagement mechanism 316 may sometimes be described herein as separate from anchoring sleeve 306, in some examples, engagement mechanism 316 may be manufactured as part of anchoring sleeve 306. In some examples, engagement mechanism 316 may be manufactured separately from anchoring sleeve 306 and be attachable to anchoring sleeve 306, e.g., placed around existing anchoring sleeve designs. In some examples, anchoring sleeve 306 may be configurable between an expanded state and a contracted state via engagement mechanism 316.

In some example, parts or all of engagement mechanism 316 may be absorbable, resorbable, or biodegradable, such that the parts or all of engagement mechanism 316 may be absorbed by the patient's body after patient tissue 314 has had time to heal.

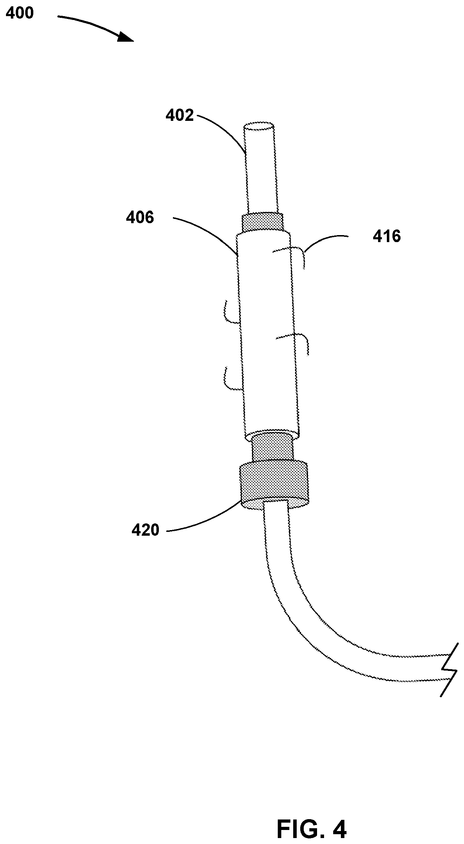

FIG. 4 is a conceptual drawing illustrating example lead anchoring system 400 including introducer 420, in accordance with one or more techniques of the disclosure. Lead anchoring system 400 also includes lead 402 and anchoring sleeve 406, which may be substantially similar to lead 102 and anchoring sleeve 106, respectively, as shown in FIG. 1. Anchoring sleeve 406 includes engagement mechanism 416, which may be substantially similar to engagement mechanism 316 as shown in FIG. 3.

Introducer 420 may be configured to deliver anchoring sleeve 406 along lead 402 to the incision site. Introducer 420 may include an inner diameter defining a lumen, wherein introducer 420 is sized to have lead 402 inserted through the introducer lumen. Introducer 420 may also include an outer diameter sized to be inserted into a lumen defined of anchoring sleeve 406 defined by an inner diameter of anchoring sleeve 406. Introducer 420 may be made of materials with low surface friction to allow anchoring sleeve 406 to slide on or off introducer 420, and to allow introducer 420 to slide along lead 402. In some examples, a lubricant may assist introducer 420 to translate along lead 402. Introducer 420 may define a suitable length to hold anchoring sleeve 406.

Anchoring sleeve 406 may be configurable between an expanded and a contracted state. In some examples, introducer 420 may hold anchoring sleeve 406 in the expanded state while anchoring sleeve 406 surrounds introducer 420. Anchoring sleeve 406 may have a first inner diameter (e.g., substantially equal to an outer diameter of introducer 420) in the expanded state and a second inner diameter in the contracted state, where the second inner diameter is smaller than the first inner diameter. Introducer 420 may deliver anchoring sleeve 406 in the expanded state along lead 402 to the incision site. In some examples, anchoring sleeve 406 is removed from introducer 420 and attached to lead 402 before engagement mechanism 416 engages with patient tissue. In some examples, anchoring sleeve 406 is removed from introducer 420 and attached to lead 402 after engagement mechanism 416 engages with patient tissue. In examples where anchoring sleeve 406 is configured to self-collapse, anchoring sleeve 406 may self-collapse and attach to lead 402 upon removal of introducer 420, transitioning anchoring sleeve 406 to the contracted state.

When anchoring sleeve 406 collapses, engagement mechanism 416, which is engaged to patient tissue, may pull patient tissue inward in the direction of contraction. This may at least partially close the incision in patient tissue at the incision site. That is - in the expanded state - anchoring sleeve 406 and engagement mechanism 416 may define a first outer diameter, and - in the contracted state - anchoring sleeve 406 and engagement mechanism 416 may define a second outer diameter. The second outer diameter may be smaller than the first outer diameter. Engagement mechanism 416 may engage with patient tissue substantially at or near the outer diameter of anchoring sleeve 406.

For example, anchoring sleeve 406 may be made at least partially of a polymeric material configured to expand and contract. The polymeric material of anchoring sleeve 406 may be held in a high-energy state (expanded) when surrounding introducer 420, and define a first inner diameter of anchoring sleeve 406 and a first outer diameter of anchoring sleeve 406. Introducer 420 may deliver anchoring sleeve 406 to an incision site in a patient, where engagement mechanism 416 of anchoring sleeve 406 may engage with patient tissue. Engagement mechanism 416 may engage with patient tissue on either sides of an incision at the incision site, such that anchoring sleeve 406 is positioned at least partially within the incision and between the severed tissue of the patient. Introducer 420 may be removed. For example, introducer 420 and anchoring sleeve 406 may travel distally along lead 402 to the incision site, and once engagement mechanism 416 is attached to patient tissue, introducer 420 may be withdrawn proximally along lead 402. Anchoring sleeve 406 may be held in place while introducer 420 is withdrawn, either by attachment to the patient tissue via engagement mechanism 416, or for example by a finger or other tool of a physician. In this way introducer 420 may be removed from an inner lumen of anchoring sleeve 406, allowing the polymeric material of anchoring sleeve 406 to transition to a low-energy state (contract) onto lead 402. After contracting, the inner diameter of anchoring sleeve 406 may shrink to a second inner diameter, and the outer diameter of anchoring sleeve 406 may shrink to a second outer diameter. Because anchoring sleeve 406 is attached to patient tissue via engagement mechanism 416 near or substantially at the outer diameter of anchoring sleeve 406, the patient tissue may be drawn together in the direction of the contraction of anchoring sleeve 406. As the patient tissue is drawn together around anchoring sleeve 406, the incision at least partially closes.

In some examples, anchoring sleeve 406 may be made at least partially of shape-memory or elastic metal alloys that allow anchoring sleeve 406 to expand and contract. The metal alloys may exist in a high-energy state when anchoring sleeve 406 is in the expanded state, and may self-collapse into a low-energy state to transition to the contracted state when not subject to an expanding force. In some examples, the expanded state and contracted state of anchoring sleeve 406 may not define a high-energy and a low-energy state. Anchoring sleeve 406 may transition between the expanded state and the contracted state through mechanical means when different forces are applied.

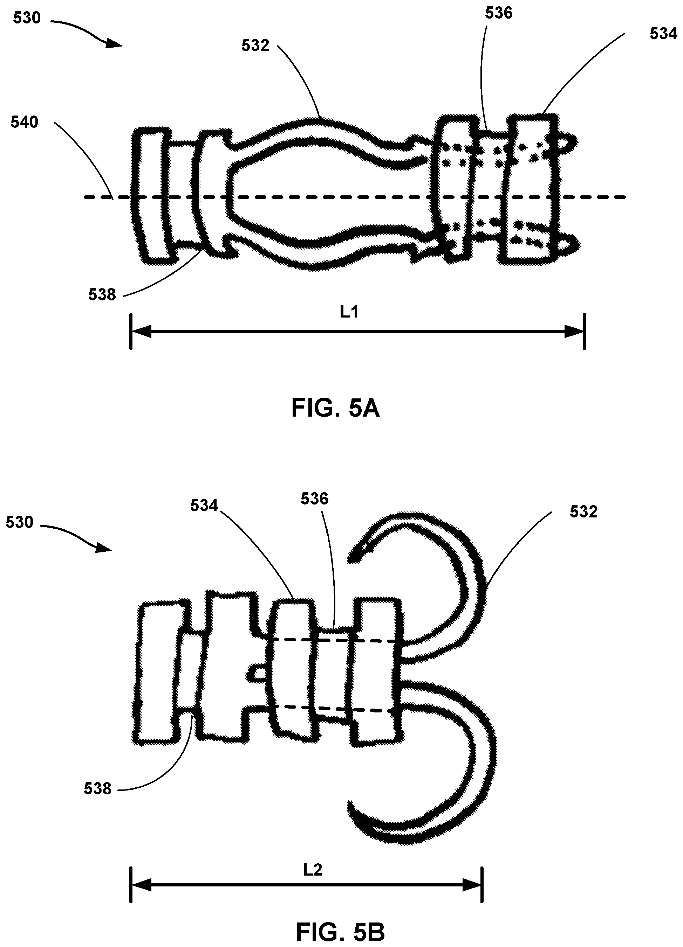

FIG. 5A is a conceptual drawing illustrating an example tine assembly 530 in a constrained state, in accordance with one or more techniques of the disclosure. FIG. 5B is a conceptual drawing illustrating the example tine assembly 530 of FIG. 5A in an unconstrained state, in accordance with one or more techniques of the disclosure. In some examples, tine assembly 530 may be a part of an engagement mechanism of an anchoring sleeve. Tine assembly 530 may allow the engagement mechanism to attach to patient tissue at an incision site in a patient.

As shown in FIGS. 5A-5B, tine assembly 530 may include a plurality of tines 532 configured to transition between a constrained state and an unconstrained state. Although only two tines 532 are shown as part of tine assembly 530, tine assembly 530 may include any number of one or more tines. Transitioning from the constrained state to the unconstrained state may cause the plurality of tines to engage with the patient tissue and assist closure of an incision in the patient tissue at the incision site. Tines 532 may be made of one or more metal alloys, e.g., nitinol, that allow them to elastically deform. Tines 532 may be in a high-energy state when in the constrained state, and may be in a low-energy state in the unconstrained state, such that, absent a force holding tines 532 in the constrained state, tines 532 may automatically transition to the unconstrained state.

As shown in FIG. 5A, two or more tines 532 may extend substantially along the direction of longitudinal axis 540 of tine assembly 530 when the two or more tines 532 are in the constrained state. Longitudinal axis 540 may pass through a center-point of tine assembly 430. Compression fitting 534 may hold the two or more tines 532 in the constrained state. For example, the two or more tines 532 may exert two or more forces against an interior lumen of compression fitting 534 in the constrained state that cancel one another out when the forces of each tine of the two or more tines 532 are combined. The forces due to the two or more tines 532 may only cancel out in an axis perpendicular to the shown longitudinal axis 540, while small inequalities in forces along longitudinal axis 540 due to the two or more tines 532 in the constrained state may be resisted by friction between the two or more tines 532 and compression fitting 534. Each of the two or more tines 532 may be configured to bend radially away from longitudinal axis 540. Although compression fitting 534 is shown in FIGS. 5A-5B as centered along longitudinal axis 540, in some examples the forces exerted by each tine of tines 532 may balance when compression fitting 534 is slightly further from longitudinal axis 540 in one radial direction away from longitudinal axis 540 than another. Tine assembly 530 may define a constrained length L₁ in the constrained state.

Compression fitting 534 may be configured to slide along tines 532. For example, an internal lumen of compression fitting 534 may be made of relatively low friction materials, such that when compression fitting 534 is acted on by an outside force along longitudinal axis 540, compression fitting 534 may slide along tines 532 in one or another longitudinal direction. Compression fitting 534 may include one or more surface features (e.g., first surface feature 536) to allow a tool to grasp compression fitting 534. As shown in the example of FIGS. 5A-5B, first surface feature 536 is located on an exterior of compression fitting 534, although it may be located anywhere on compression fitting 534. The two or more tines 532 may also include or be attached to one or more surface features (e.g., second surface feature 538) to allow a tool to grasp a body of tine assembly 530 or otherwise hold the two or more tines 532 in place while sliding compression fitting 534 along the two or more tines 532.

When compression fitting 534 slides along different portions of the two or more tines 532, e.g., towards the unconstrained state shown in FIG. 5B, it may cause the two or more tines 532 to transition between the constrained state and the unconstrained state. For example, when compression fitting 534 is positioned around one end of tines 532 as shown in FIG. 5A, tines 532 may be held in a substantially straight configuration. When compression fitting 534 is slid along tines 532 to a different position as shown in FIG. 5B, tines 532 may no longer be constrained by compression fitting 534 and may curve into a low-energy state, the unconstrained state. Tine assembly 530 may define an unconstrained length L₂ in the unconstrained state. In some examples the unconstrained length L₂ may be smaller than the constrained length L₁ of tine assembly 530.

Tines 532 may be configured to engage with patient tissue or disengage with patient tissue. For example, when tines 532 transition from the constrained state to the unconstrained state, they may engage with patient tissue. When tines 532 are in the constrained state, compression fitting 534 may prevent the tips of tines 532 from engaging with patient tissue. When compression fitting 534 slides along longitudinal axis 540 of tine assembly 530 to transition tines 532 to the unconstrained state, the tips of tines 532 may then be allowed to pierce into patient tissue.

For example, a physician may press tine assembly 530 in a constrained state against patient tissue such that longitudinal axis 540 is perpendicular to a plane defined by patient tissue, and wherein the tips of tines 532 are in contact with the patient tissue. Because tines 532 are in the constrained state, they do not engage with patient tissue. The physician may continue applying pressure against patient tissue with tine assembly 530 while sliding compression fitting 534 along longitudinal axis 540 such that tines 532 transition to the unconstrained state. In some examples, the physician may grip tines 532 and/or the compression fitting using surface features 536, 538. Compression fitting 534 may slide along longitudinal axis 540 away from the contact point between tines 532 and patient tissue. As compression fitting 534 slides along longitudinal axis 540, the tips of tines 532 may be allowed to pierce into patient tissue and anchor tine assembly 530 to the patient tissue.

Because the unconstrained length L₂ may be smaller than the constrained length L₁ of tine assembly 530, when tines 532 engage with patient tissue, they may also pull patient tissue in the direction of the length of tine assembly 530. In some examples, an anchoring sleeve may include one or more of tine assemblies, each with one or more tines 532, where the tips of the one or more tines 532 extend radially outward from the anchoring sleeve. When tines 532 transition from the constrained state to the unconstrained state and engage with patient tissue at an incision site, tines 532 may pull the patient tissue inward towards the anchoring sleeve and at least partially close an incision in the patient tissue at the incision site.

FIG. 6 is a conceptual drawing illustrating an example anchoring sleeve 606 including a coiling tine 632 to engage with patient tissue, in accordance with one or more techniques of the disclosure. Coiling tine 632 may be a part of engagement mechanism 616.

In some examples, engagement mechanism 616 may be configured to completely surround coiling tine 632 while anchoring sleeve 606 is being delivered to an incision site in patient tissue. Once anchoring sleeve 606 is positioned in an incision in the patient tissue, engagement mechanism 616 may be configured to be rotated to allow coiling tine 632 to extend out of engagement mechanism 616 such that at least part of coiling tine 632 is no longer surrounded by engagement mechanism 616. Although only one coiling tine 632 is shown in FIG. 6, in some examples, engagement mechanism 616 includes multiple coiling tines. In some examples, anchoring sleeve 606 may be configured to be rotated to allow coiling tine 632 to extend out of engagement mechanism 616. In some examples, one or both of anchoring sleeve 606 and engagement mechanism 616 may be configured to rotate in one direction or opposite directions to allow coiling tine 632 to extend out of engagement mechanism 616.

The tip of coiling tine 632 may be configured to engage with patient tissue when it extends out of engagement mechanism 616. As coiling tine 632 extends further out of engagement mechanism 616, it may continue to pierce into patient tissue. Patient tissue may be forced to twist along coiling tine 632, such that patient tissue is constricted around anchoring sleeve 606. In this way, coiling tine 632 may at least partially close the incision in patient tissue at the incision site.

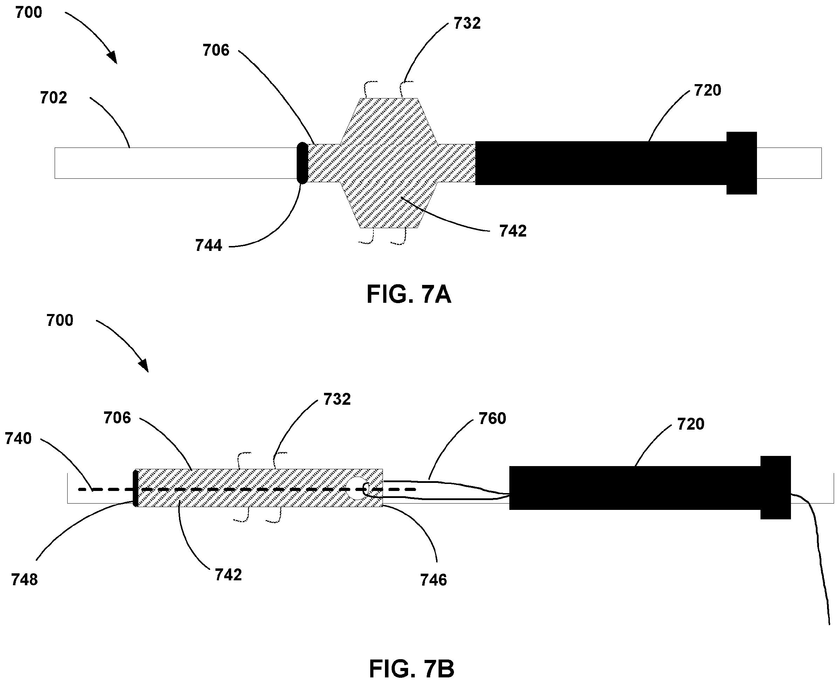

FIG. 7A is a conceptual drawing illustrating an example lead anchoring system 700 including an expanding feature 742 in an expanded state, in accordance with one or more techniques of the disclosure. FIG. 7B is a conceptual drawing illustrating the example lead anchoring system of FIG. 7A, including expanding feature 742 in a contracted state, in accordance with one or more techniques of the disclosure. System 700 includes lead 702, anchoring sleeve 706 and introducer 720. Anchoring sleeve 706 may include an engagement mechanism (e.g., expanding feature 742), and friction mechanism 744. Expanding feature 742 may include an attachment mechanism (e.g., tines 732) to attach expanding feature 742 with patient tissue. System 700, lead 702, and anchoring sleeve 706 may be substantially similar to system 100, lead 102, and anchoring sleeve 106, respectively of FIG. 1. Introducer 720 may be substantially similar to introducer 420 of FIG. 4.

Anchoring sleeve 706 may be configurable between an expanded and a contracted state via expanding feature 742. Expanding feature 742 may be in a high-energy state when in the expanded state (as shown in FIG. 7A), and in a low-energy state when in the contracted state (as shown in FIG. 7B), such that expanding feature 742 self-collapses to transition to the contracted state when not subject to an outside force. For example, expanding feature 742 may be configured to transition between the expanded and contracted state in response to a compressive force along longitudinal axis 740 of anchoring sleeve 706. Longitudinal axis 740 may be defined by a length between distal end 748 and proximal end 746 of anchoring sleeve 706. Expanding feature 742 may be configured to transition to the expanded state when subject to the compressive force, and may self-collapse to the contracted state when the compressive force is removed.

In the example of FIGS. 7A-7B, anchoring sleeve 706 is anchored to lead 702 at least by friction mechanism 744 at distal end 748 of anchoring sleeve 706, which may prevent anchoring sleeve 706 from translating along lead 702 when subject to a force along longitudinal axis 740. In some examples, anchoring sleeve 706 does not include friction mechanism 744, and instead a finger or other tool may be used to hold distal end 748 of anchoring sleeve 706 in place. In some examples, as shown in FIG. 7A, introducer 720 may provide a compressive force to proximal end 746 of anchoring sleeve 706, compressing anchoring sleeve 706 with respect to longitudinal axis 740 and holding anchoring sleeve 706 in the expanded state. When introducer 720 does not apply a compressive force to proximal end 746, as shown in FIG. 7B, anchoring sleeve 706 may automatically extend with respect to longitudinal axis 740 and transition to the contracted state.

Tines 732 may be configured to engage with patient tissue. Although described as tines, in some examples, expanding feature 742 may include one or more types of attachment mechanism to engage with patient tissue, e.g., adhesive. Tines 732 may engage with patient tissue at an incision site while anchoring sleeve 706 is in the expanded state. When anchoring sleeve 706 transitions from the expanded state to the contracted state, tines 732 may pull patient tissue towards anchoring sleeve 706, assisting closure of an incision at the incision site.

In some examples, expanding feature 742 may not self-collapse from the expanded state to the contracted state once no longer acted on by a compressive force in the direction of longitudinal axis 740. In some examples, expanding feature 742 may be configured to attach to tether 760. Tether 760 may be configured to apply a tension force to expanding feature 742 in the direction of longitudinal axis 740. For example, tether 760 may be attached at one end of tether 760 to proximal end 746 of expanding feature 742, and at another end of tether 760 to introducer 720. When introducer 720 is retracted from the incision site (e.g., translated proximally along lead 702 away from the incision site), introducer 720 may pull on tether 760, which in turn may pull on proximal end 746 of anchoring sleeve 706, causing anchoring sleeve 706 to transition from the expanded state to the contracted state.

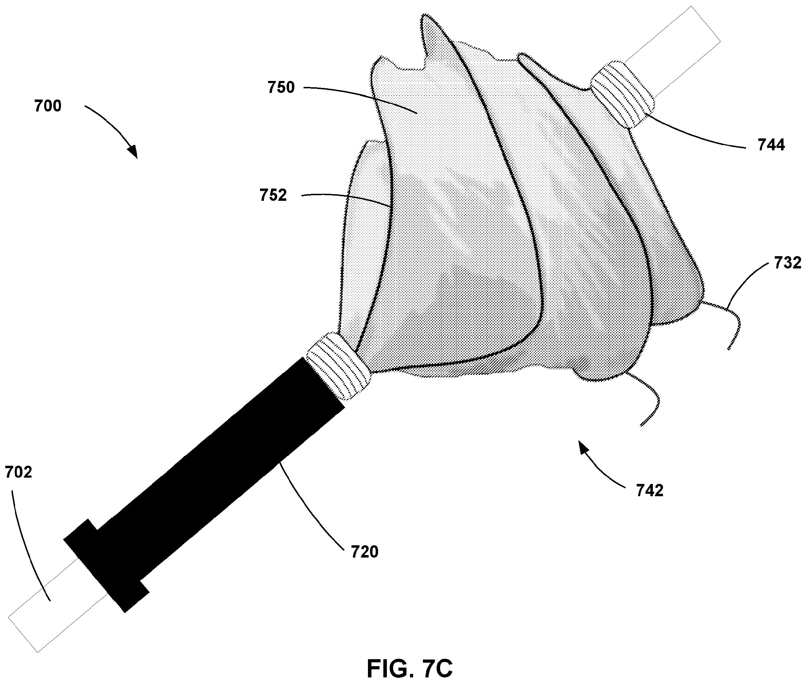

FIG. 7C is a conceptual drawing illustrating example lead anchoring system 700 of FIGS. 7A and 7B including one or more elastic spines 752, in accordance with one or more techniques of the disclosure. Expanding feature 742 may include one or more spines 752 and envelope 750 stretching between each of the one or more spines 752.

Expanding feature 742 may be configurable between an expanded state and a contracted state via the one or more spines 752 and envelope 750. For example, the one or more spines 752 may be configured to expand envelope 750 when the one or more spines 752 are subject to a compressive force along the longitudinal axis of the anchoring sleeve, as discussed with respect to FIGS. 7A-7B. For example, Spines 752 may be made of elastic metal or metal alloy, and coiled around a length of the anchoring sleeve. Envelope 750 may be made of a flexible polymer or fabric.

Expanding feature 742 may also include one or more tines 732 configured to engage with patient tissue at an incision site. Tines 732 may engage with patient tissue at the incision site when expanding feature 742 is in the expanded state. When expanding feature 742 transitions from the expanded state to the contracted state, the transition may cause expanding feature 742 to assist closure of an incision in the patient tissue at the incision site. Although only two tines 732 are shown in FIG. 7C, in some examples, tines 732 may include any number of tines attached at any number of points on spines 752 and/or envelope 750.

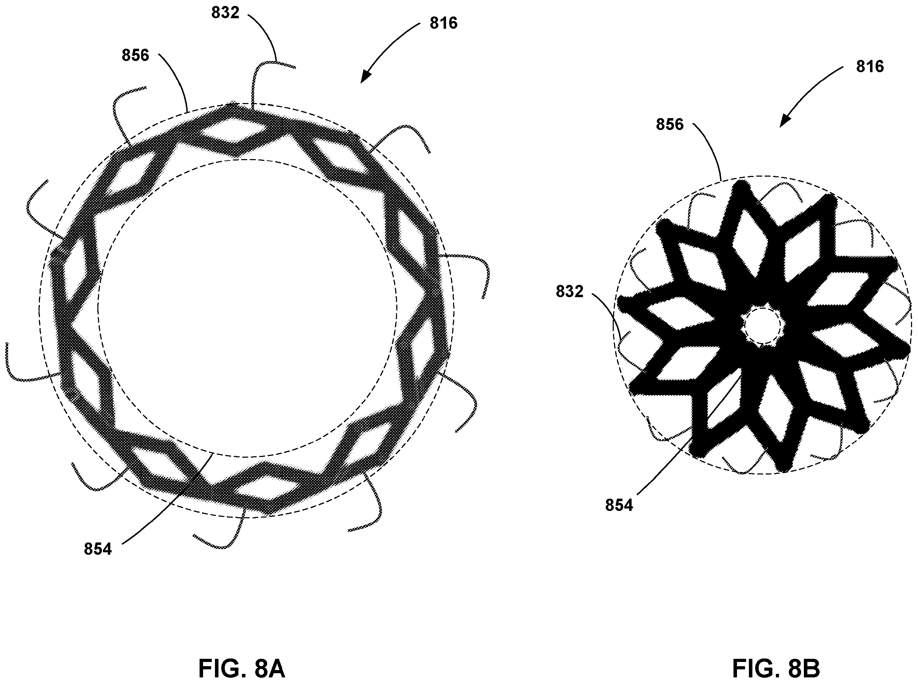

FIG. 8A is a conceptual drawing illustrating an example strut assembly 816 of an anchoring sleeve in an expanded state, in accordance with one or more techniques of the disclosure. FIG. 8B is a conceptual drawing illustrating example strut assembly 816 of FIG. 8A in a contracted state, in accordance with one or more techniques of the disclosure. In some examples, the engagement mechanism includes strut assembly 816. Strut assembly 816 may include a plurality of struts that define outer diameter 856 and inner diameter 854. Strut assembly may also include a plurality of tines 832 attached to and extending from the plurality of struts defining outer diameter 856. One or more of the plurality of struts may be attached to one another through rotatable connections.

Strut assembly 816 may be configurable between an expanded state and a contracted state. Outer diameter 856 in the expanded state may be larger than outer diameter 856 in the contracted state. Similarly, inner diameter 854 in the expanded state may be larger than inner diameter 854 in the contracted state. The plurality of tines 832 may be configured to engage with patient tissue at an incision site while strut assembly 816 is in the expanded state. When strut assembly 816 transitions from the expanded state to the contracted state, the transition may cause the plurality of tines to at least partially close an incision in the patient tissue at the incision site. As shown in FIGS. 8A-8B, tines 832 contract inward towards the center of strut assembly 816 when strut assembly 816 transitions from the expanded state to the contracted state. When tines 832 are engaged with patient tissue at an incision site, they may pull the incision at least partially closed at the diameters of strut assembly 816 reduce. Although described with respect to FIGS. 8A-8B as including a plurality of tines 832, in some examples strut assembly 816 includes one or more methods for engaging with patient tissue, e.g., adhesive.

In some examples, strut assembly 816 may be made at least partially of shape-memory or elastic metal alloys that strut assembly 816 to expand and contract. The metal alloys may exist in a high-energy state when strut assembly 816 is in the expanded state, and may self-collapse into a low-energy state to transition to the contracted state when not subject to an expanding force. In some examples, strut assembly 816 may not be configured to self-collapse, and may transition between the expanded and contracted state in response to force applied to strut assembly 816 from an external source, e.g., physician's fingers. The rotatable connections between each strut of the plurality of struts in strut assembly 816 may allow a physician to easily transition strut assembly 816 to an expanded state by pulling on substantially opposing sides of strut assembly 816 in a radial direction away from a center of strut assembly 816. The rotatable connections between each strut may also allow a physician to transition strut assembly 816 to a contracted state by applying a force inward on substantially opposing sides in a radial direction towards the center of strut assembly 816.

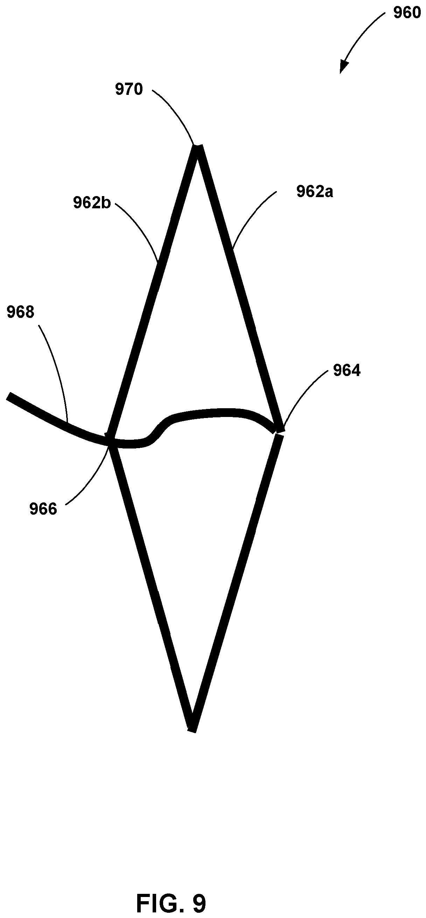

In some examples, a fastener may be rigidly attached to a first side of strut assembly 816, e.g., at any point on inner diameter 854, and may be slidably attached to a second side of strut assembly 816, such that when a force is applied to the fastener, the force is translated to the rigid attachment, and allows strut assembly 816 to transition from the expanded state to the contracted state. For example, a physician may engage strut assembly 816 in patient tissue at an incision site when strut assembly 816 is in an expanded state, then pull on the fastener to transition strut assembly 816 from the expanded state to the contracted state. This procedure is described on a smaller scale with reference to FIG. 9 below. Although the fastener shown in FIG. 9 is attached to opposing sides of a single parallelogram of strut assembly 816, the fastener may be attached at a multitude of combinations of points on strut assembly 816 that allow a pulling force on the fastener to transition strut assembly 816 from the expanded state to the contracted state.

FIG. 9 is a conceptual drawing illustrating an example tie mechanism 960 for a strut assembly, in accordance with one or more techniques of the disclosure. Tie mechanism 960 may be included as part of a strut assembly of an engagement mechanism, for example strut assembly 816 of FIGS. 8A-8B. Tie mechanism 960 may include a plurality of struts 962, wherein each strut of the plurality of struts 962 rotatably connects (e.g., via rotatable connection 970) to at least one other strut of the plurality of struts 962.

Tie mechanism 960 may also include fastener 968. The plurality of struts 962 of tie mechanism 960 may define a parallelogram. Fastener 968 may be rigidly attached to a first side of the parallelogram defined by the plurality of struts 962, e.g., at rigid attachment point 964. Fastener 968 may be slidably attached to a second side of the parallelogram defined by the plurality of struts 962, e.g., at slidable attachment point 966. Fastener 968 may apply a pulling force to rigid attachment point 964 when fastener 968 is subject to a pulling force. While the pulling force pulls rigid attachment point 964, fastener 968 may be allowed to slide past slidable attachment point 966, pulling the first side of the parallelogram to the second side of the parallelogram, and closing the parallelogram. When the parallelogram defined by the plurality of struts 962 is only a first of a plurality of parallelograms that make up a strut assembly (e.g., strut assembly 816 of FIGS. 8A-8B), the force pulling on the first parallelogram may be translated among all the parallelograms of the strut assembly to transition the strut assembly between an expanded and a contracted state.

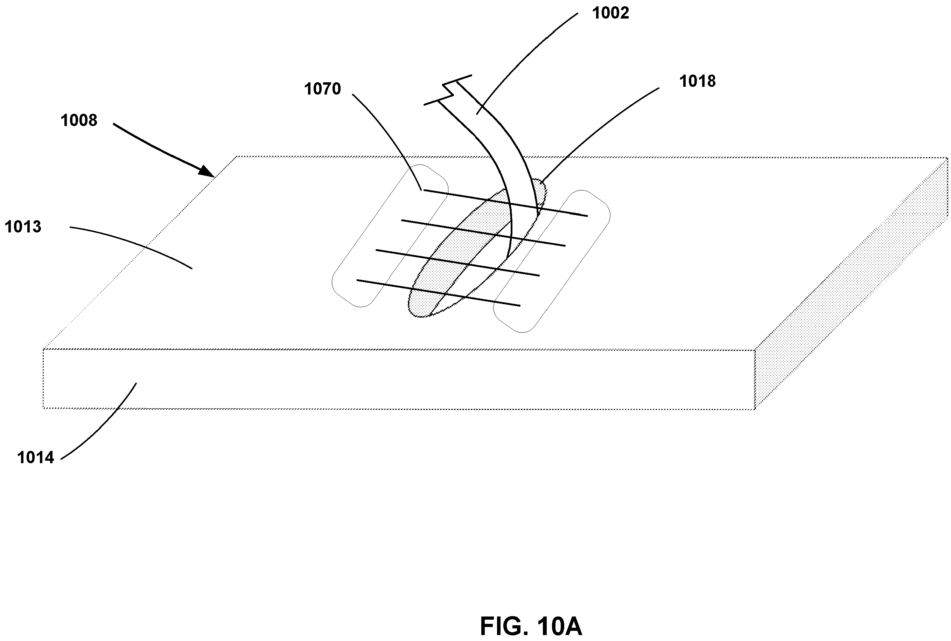

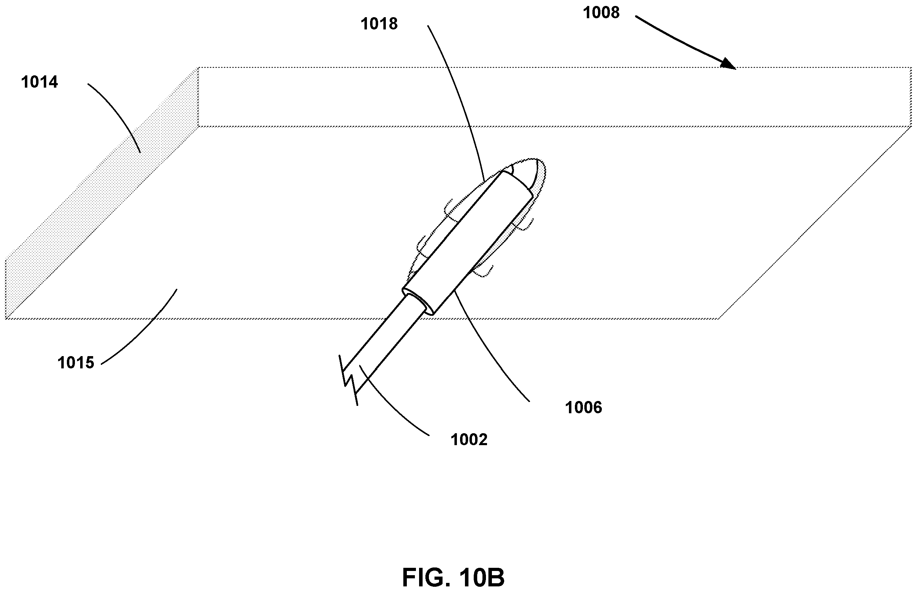

FIG. 10A is a conceptual drawing illustrating an example outer side 1013 of patient tissue 1014 at an incision site 1008, in accordance with one or more techniques of the disclosure. FIG. 10B is a conceptual drawing illustrating an example inner side 1015 of patient tissue 1014 at incision site 1008 of FIG. 10A, in accordance with one or more techniques of the disclosure. Anchoring sleeve 1006 may be disposed substantially within incision 1018 on lead 1002.

In some examples, anchoring sleeve 1006 may be paired with other techniques for closing incision wounds in the patient. In some examples, anchoring sleeve 1006 may be configured to engage (e.g., via an engagement mechanism) with inner side 1015 of patient tissue 1014 at incision site 1008 and assist closure of incision 1018. In some examples, a closure device 1070 may be configured to engage with outer side 1013 of patient tissue 1014 at incision site 1008 and assist closure of incision 1018.

In some examples, anchoring sleeve 1006 may assist closure of a first incision in first patient tissue, while closure device 1070 may assist closure of a second incision in second patient tissue, wherein the first incision is deeper within the patient's body than the second incision. For example, the first incision may be in interior bodily tissue of the patient, e.g., the mediastinal pleurae, the fascia covering the pectoralis major muscle, or whatever may form the pocket floor during an implant procedure. In some examples, the second incision may be in skin tissue of the patient above the first incision.

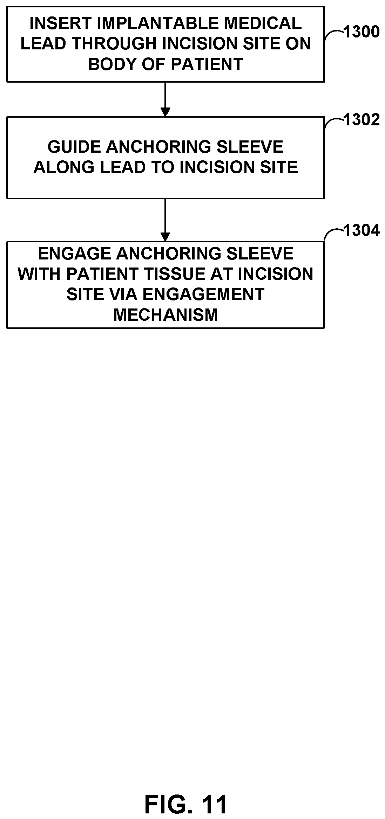

FIG. 11 is a flow diagram of an example technique for closing an incision in patient tissue using an anchoring sleeve configured to assist closure of the incision, in accordance with one or more techniques of the disclosure. The example technique of FIG. 11 may be used with any of the leads, anchoring sleeves, and attachment mechanisms described with respect to FIGS. 1-10.

The example technique may include inserting an implantable medical lead of a medical device system through an incision site on a body of a patient (1300). The medical device system may include an implantable medical lead configured to be delivered through an incision site on a body of a patient and an anchoring sleeve surrounding a portion of the lead. Components of the medical device system may be utilized with various types of implant tool systems, such as implant tool systems for delivering IMDs configured to deliver electrical therapy (e.g., cardiac electric therapy, neurostimulation), or other implant tool systems. Furthermore, although primarily described herein as being an implantable medical lead (e.g., a cardiac lead), the lead may be another type of device, such as a catheter.

The lead may include a proximal end and a distal end. In some examples the distal end may include one or more electrodes and rest against or be attached to patient tissue within the patient. In some examples the proximal end may be connected to a medical device, for example an implantable cardiac defibrillator (ICD). In some examples, the medical device is configured to be implanted in the body of the patient. In some examples, the medical device may remain on the exterior of the patient with only the lead entering the patient. Although the technique is described with reference to only one lead, in some examples, the medical device system includes multiple leads and multiple anchoring sleeves for each of the leads.

In some examples, the lead may be entirely implanted within the patient. For example, the lead may be connected to an ICD that is subcutaneously implanted on the left midaxillary of the patient, where the lead extends subcutaneously from the ICD to a treatment site within the patient. In some examples, the incision site is not an incision in the skin, but in interior bodily tissue of the patient, e.g., the mediastinal pleurae, the fascia covering the pectoralis major muscle, or whatever may form the pocket floor during an implant procedure. In some examples, the incision site includes incision through both skin tissue of the patient as well as other connective tissues of the patient.

In some examples, a clinician may insert the lead into and through a patient's vasculature to a target site within a body of the patient (e.g., tissue of a heart of the patient) where a medical procedure may be undertaken. In some examples, the clinician may insert the lead under the patient's sternum and to a target site within the body of the patient, rather than through the vasculature. The incision site may be located anywhere on the body of the patient. The lead may be temporary (e.g., the lead may be a temporary pacing balloon lead) or permanent. The length of the lead may vary.

The technique may also include making a first incision through skin of the patient at an incision site, for example to form a pocket in the patient tissue for implantation of an IMD connected to the lead. A second incision may allow the lead to extend from the pocket and further into the body of the patient. In some examples, once implanted, the lead may extend subcutaneously from the implanted IMD toward the xiphoid process. At a location near the xiphoid process the lead may bend or turn and extend superior upward in the substernal space. In one example, the lead may be placed in the mediastinum and, more particularly, in the anterior mediastinum. The anterior mediastinum is bounded laterally by pleurae, posteriorly by the pericardium, and anteriorly by the sternum. In some examples, the lead may be implanted within the mediastinum such that one or more electrodes of the lead are located over a cardiac silhouette of the ventricle as observed via fluoroscopy. In some examples, the lead may be located substantially centered under the sternum. In other examples the lead may be implanted such that it is offset laterally from the center of the sternum. Although described herein as being implanted in the substernal space, the mediastinum, or the anterior mediastinum, the lead may be implanted in other extra-pericardial locations.

The example technique may further include guiding the anchoring sleeve of the medical device system to the incision site, wherein the anchoring sleeve surrounds a portion of the lead (1302). The anchoring sleeve may be configured to anchor the lead in place at the incision site. That is, the anchoring sleeve may prevent the lead from shifting or moving once fixed to tissue at the incision site. The anchoring sleeve may be configured to attach to the lead through any method known in the art.

In some examples, the medical device system also includes an introducer. The introducer may be configured to deliver the anchoring sleeve along the lead to the incision site. Thus, the technique may include guiding the introducer along the lead to the incision site. The technique may also include removing the anchoring sleeve from the introducer at or near the incision site, and removing the introducer from the incision site. For example, the introducer may translate proximally along the lead away from the incision site and eventually removed from the lead.

The introducer may include an inner diameter defining a lumen, wherein the introducer is sized to have the lead inserted through the introducer lumen. The introducer may also include an outer diameter sized to be inserted into a lumen of the anchoring sleeve defined by an inner diameter of the anchoring sleeve. The introducer may be made of materials with low surface friction to allow the anchoring sleeve to slide on or off the introducer, and to allow the introducer to slide along the lead. In some examples, a lubricant may assist the introducer to translate along the lead. The introducer may define a suitable length to hold the anchoring sleeve.

The anchoring sleeve may be configurable between an expanded and a contracted state. In some examples, the introducer may hold the anchoring sleeve in the expanded state while the anchoring sleeve surrounds the introducer. The anchoring sleeve may have a first inner diameter (e.g., substantially equal to an outer diameter of the introducer) in the expanded state and a second inner diameter in the contracted state, where the second inner diameter is smaller than the first inner diameter. The introducer may deliver the anchoring sleeve in the expanded state along the lead to the incision site. In some examples, the technique includes removing the anchoring sleeve from the introducer and attaching the anchoring sleeve to the lead before engaging the engagement mechanism with patient tissue. In some examples, the technique includes engaging the engaging mechanism with patient tissue before removing the anchoring sleeve from the introducer and attaching the anchoring sleeve to the lead. In examples where the anchoring sleeve is configured to self-collapse, the anchoring sleeve may self-collapse and attach to the lead upon removal of the introducer, transitioning the anchoring sleeve to the contracted state.

When the anchoring sleeve collapses, the engagement mechanism, which is engaged to patient tissue, may pull patient tissue inward in the direction of contraction. This may at least partially close the incision in patient tissue at the incision site. That is - in the expanded state - the anchoring sleeve and the engagement mechanism may define a first outer diameter, and - in the contracted state - the anchoring sleeve and the engagement mechanism may define a second outer diameter. The second outer diameter may be smaller than the first outer diameter. The engagement mechanism may engage with patient tissue substantially at or near the outer diameter of the anchoring sleeve.

For example, the anchoring sleeve may be made at least partially of a polymeric material configured to expand and contract. The polymeric material of the anchoring sleeve may be held in a high-energy state (expanded) when surrounding the introducer, and define a first inner diameter of the anchoring sleeve and a first outer diameter of the anchoring sleeve. The technique may include delivering the anchoring sleeve to an incision site in a patient using the introducer, where the engagement mechanism of the anchoring sleeve may engage with patient tissue. The engagement mechanism may engage with patient tissue on either sides of an incision at the incision site, such that the anchoring sleeve is positioned at least partially within the incision and between the severed tissue of the patient. The introducer may be removed. For example, the introducer and the anchoring sleeve may travel distally along the lead to the incision site, and once the engagement mechanism is attached to patient tissue, the introducer may be withdrawn proximally along the lead. The anchoring sleeve may be held in place while the introducer is withdrawn, either by attachment to the patient tissue via the engagement mechanism, or for example by a finger or other tool of a physician. In this way the introducer may be removed from an inner lumen of the anchoring sleeve, allowing the polymeric material of the anchoring sleeve to transition to a low-energy state (contract) onto the lead. After contracting, the inner diameter of the anchoring sleeve may shrink to a second inner diameter, and the outer diameter of the anchoring sleeve may shrink to a second outer diameter. Because the anchoring sleeve is attached to patient tissue via the engagement mechanism near or substantially at the outer diameter of the anchoring sleeve, the patient tissue may be drawn together in the direction of the contraction of the anchoring sleeve. As the patient tissue is drawn together around the anchoring sleeve, the incision at least partially closes.

In some examples, the anchoring sleeve may be made at least partially of shape-memory or elastic metal alloys that allow the anchoring sleeve to expand and contract. The metal alloys may exist in a high-energy state when the anchoring sleeve is in the expanded state, and may self-collapse into a low-energy state to transition to the contracted state when not subject to an expanding force. In some examples, the expanded state and contracted state of the anchoring sleeve may not define a high-energy and a low-energy state. The anchoring sleeve may transition between the expanded state and the contracted state through mechanical means when different forces are applied.

The anchoring sleeve may be positioned partially in the subcutaneous tissue and partially within the substernal space. The anchoring sleeve may be coupled to the patient tissue at one or more points along the length of the anchoring sleeve.

In some existing procedures, a physician must couple an anchoring sleeve to a patient via one or more sutures that are sewn through patient tissue at an incision site and tied or otherwise wrapped around the anchoring sleeve. Significant force must be applied to the sutures to tighten them and hold the anchoring sleeve and patient tissue together. This procedure can be difficult, as the physician is attempting to manipulate the sutures in a moist environment through a small hole some depth within patient tissue (e.g., three inches). In order to reduce the time and difficulty of the lead implant procedure, the anchoring sleeve may include features to engage with patient tissue and assist closure of the incision.

By incorporating the anchoring sleeve into the medical device system, wherein the anchoring sleeve and engagement mechanism assist closure of the incision, physicians may be saved the time and difficulty of performing other attachment methods.

The example technique may further include engaging the anchoring sleeve with patient tissue at the incision site via an engagement mechanism of the anchoring sleeve, wherein the engagement mechanism is configured to assist closure of an incision in the patient tissue at the incision site (1304).

The anchoring sleeve may include an engagement mechanism to engage with patient tissue. The engagement mechanism may include, for example, one or more elongated tines such as fixation tines configured to substantially maintain an orientation of the lead with respect to the incision site. In some examples, the engagement mechanism may include adhesives or other mechanism for engaging with patient tissue. In some examples, the engagement mechanism may be attached to the anchoring sleeve, or otherwise manufactured as a part of the anchoring sleeve. In some examples, the engagement mechanism may be retractable/advanceable/assembled onto a separate anchoring sleeve before implantation of the lead.

In some examples, the anchoring sleeve may be configurable between an expanded and a contracted state, by way of the engagement mechanism or separate from the functioning of the engagement mechanism. When the anchoring sleeve transitions from the expanded to the contracted state, the anchoring sleeve may assist closure of the incision in the patient tissue.

The engagement mechanism may be configured to engage with patient tissue at the incision site while the anchoring sleeve is disposed within the incision. The engagement mechanism may engage with the severed portions of the patient tissue representing the inner walls defined by the incision while the anchoring sleeve is in the expanded state. When the anchoring sleeve transitions to the contracted state, the engagement mechanism may pull the incision at least partially closed by pulling the inner walls of the incision towards one another.

In some examples, the anchoring sleeve and/or engagement mechanism may be made of shape memory alloys and or polymeric structures that can expand and be self-collapsing. The anchoring sleeve and engagement mechanism may be held in the expanded state while being navigated to the incision site and engaged with patient tissue. Once the engagement mechanism is attached to the patient tissue, the anchoring sleeve and engagement mechanism may be allowed to self-collapse to transition to the contracted state, pulling the incision at least partially closed. In some examples the anchoring sleeve and/or engagement mechanism may be collapsed through a mechanically actuated force.

In some example, parts or all of the engagement mechanism may be absorbable, resorbable, or biodegradable, such that the parts or all of the engagement mechanism may be absorbed by the patient's body after the patient tissue has had time to heal.

In some examples, the anchoring sleeve includes one or more shape-memory tines held in a high energy state. When the shape-memory tines are allowed to transition to their low-energy state, they may engage with patient tissue and pull it inwards towards the anchoring sleeve, at least partially closing the incision. In some examples, the shape memory tines are configured to transition between a constrained state (high-energy) and an unconstrained state (low-energy). The plurality of tines may be part of one or more tine assemblies, which themselves may be a part of an engagement mechanism of an anchoring sleeve. The tine assemblies may allow the engagement mechanism to attach to patient tissue at an incision site in a patient. The technique may include pressing the tine assembly in a constrained state against patient tissue such that a longitudinal axis of the tine assembly is perpendicular to a plane defined by patient tissue, and wherein the tips of the plurality of tines are in contact with the patient tissue. The technique may further include applying continuous pressure against patient tissue with the tine assembly while sliding the compression fitting along the longitudinal axis such that the tines transition to the unconstrained state, engaging with patient tissue and pulling the patient tissue inwards towards the anchoring sleeve, at least partially closing the incision. In some examples, the method may include gripping the tines and/or the compression fitting using one or more surface features.

In some examples, the anchoring sleeve includes an expanding feature, where an outer surface of the expanding feature includes tines configured to engage with patient tissue. The expanding feature may engage with patient tissue at the incision site when the anchoring sleeve is in the expanded state, and may pull patient tissue together when transitioned to the contracted state. The technique may include attaching a distal end of the anchoring sleeve to the implantable medical lead via a friction mechanism. The technique may also include applying a compressive force along a longitudinal axis of the expanding feature to expand the expanding features and cause the engagement mechanism to engage with patient tissue. The technique may further include removing the compressive force to cause the expanding feature to contract, wherein the expanding feature assists closure of the incision in patient tissue at the incision site when contracting.

In some examples, the anchoring sleeve includes a strut assembly configurable between an expanded state and a contracted state. The strut assembly may engage with patient tissue in the expanded state, and pull patient tissue together when transitioned to the contracted state. The technique may include engaging the strut assembly to patient tissue at the incision site via a plurality of tines around a circumference of the strut assembly when the strut assembly is in the expanded state. The technique may include contracting the strut assembly, wherein contracting the strut assembly assists closure of the incision in the patient tissue at the incision site. In some examples, the strut assembly may be configured to self-collapse to the contracted state when not subject to an expanding force. In some examples, the strut assembly may not be configured to self-collapse, and may transition between the expanded and contracted state in response to force applied to the strut assembly from an external source.

In some examples, the plurality of tines may all be oriented in the same direction (e.g., clockwise, counterclockwise) around an outer diameter of the strut assembly. That is, the plurality of tines may be oriented around a circumference of the strut assembly such that, when rotated in one direction, each of the plurality of tines engages with patient tissue at an incision site. The technique may include rotating the anchoring sleeve to cause the plurality of tines to engage with patient tissue at the incision site.

This disclosure includes various examples, such as the following examples.

Example 1: A medical device system including an implantable medical lead configured to be delivered through an incision site on a body of a patient and an anchoring sleeve surrounding a portion of the lead and including an engagement mechanism configured to: engage with patient tissue at the incision site; and assist closure of an incision in the patient tissue at the incision site.

Example 2: The medical device system of example 1, wherein the engagement mechanism includes a plurality of tines configured to transition between a constrained state and an unconstrained state, and wherein transitioning from the constrained state to the unconstrained state causes the plurality of tines to engage with the patient tissue and assist closure of the incision in the patient tissue at the incision site.

Example 3: The medical device system of example 2, further including one or more tine assemblies including: one or more surface features configured to allow a tool to grip the tine assembly; a compression fitting including one or more surface features configured to allow a tool to grip the compression fitting; and two or more tines of the plurality of tines, wherein: the two or more tines extend in the direction of a longitudinal axis of the tine assembly when the two or more tines are in the constrained state; and the two or more tines are configured to transition between the constrained state and the unconstrained state in response to the compression fitting sliding along the longitudinal axis of the tine assembly.

Example 4: The medical device system of any of examples 1-3, wherein the patient tissue at the incision site includes an outer side and an inner side, and wherein the engagement mechanism is configured to engage with the inner side of the patient tissue at the incision site.

Example 5: The medical device system of any of examples 1-4, further including an introducer configured to deliver the anchoring sleeve along the lead to the incision site.

Example 6: The medical device system of any of examples 1-5, wherein the anchoring sleeve is configurable between an expanded state and a contracted state, and wherein transitioning from the expanded state to the contracted state assists closure of the incision in the patient tissue at the incision site.

Example 7: The medical device system of any of examples 1-6, further including an introducer configured to deliver the anchoring sleeve along the lead to the incision site, wherein the introducer includes: a first inner diameter defining a first lumen, wherein the lead is sized to be inserted through the first lumen; and a first outer diameter including a low-friction material. The anchoring sleeve includes a second inner diameter defining a second lumen, wherein the first outer diameter of the introducer is sized to be inserted through the second lumen.

Example 8: The medical device system of example 7, wherein the anchoring sleeve is configurable between an expanded and a contracted state, and wherein the introducer holds the anchoring sleeve in the expanded state when the introducer is inserted through the second lumen.

Example 9: The medical device system of any of examples 1-8, wherein the anchoring sleeve includes an expanding feature configurable between an expanded and a contracted state, and wherein transitioning from the expanded state to the contracted state causes the expanding feature to assist closure of the incision in the patient tissue at the incision site.

Example 10: The medical device system of example 9, wherein the expanding feature includes: a distal end; a proximal end; and a length between the distal end and the proximal end, wherein the length defines a longitudinal axis, and wherein the expanding feature is configured to expand in response to a compressive force along the longitudinal axis, and the expanding feature is configured to contract when not subject to the compressive force.

Example 11: The medical device system of example 10, wherein the expanding feature further includes: a friction mechanism near the distal end wherein the friction mechanism is configured to attach the anchoring sleeve to a portion of the lead; an envelope; and one or more spines in contact with the envelope, wherein the one or more spines are configured to expand the envelope when the spines are subject to the compressive force along the longitudinal axis of the expanding feature, and wherein the one or more spines are configured to contract the envelope when not subject to the compressive force.

Example 12: The medical device system of any of examples 1-11, wherein the engagement mechanism includes: a plurality of struts, wherein the plurality of struts defines an inner diameter and an outer diameter, wherein the struts are configurable between an expanded state and a contracted state, and wherein the outer diameter defined by the plurality of struts in the expanded state is larger than the outer diameter defined by the plurality of struts in the contracted state; one or more rotatable connections between two or more struts of the plurality of struts; and a plurality of tines attached to and extending from the struts of the plurality of struts defining the outer diameter, wherein the plurality of tines is configured to engage with the patient tissue at the incision site, and wherein to assist closure of the incision in the patient tissue at the incision site, the engagement mechanism is configured to at least partially close the incision when the plurality of struts transitions from the expanded state to the contracted state.

Example 13: The medical device system of any of examples 1-12, wherein the engagement mechanism includes: a plurality of struts, wherein each strut of the plurality of struts rotatably connects to at least one other strut of the plurality of struts, and the plurality of struts defines a parallelogram; a fastener rigidly attached to a first side of the parallelogram defined by the plurality of struts and slidably attached to a second side of the parallelogram defined by the plurality of struts, wherein the plurality of struts is configured to close in response to a pulling force applied to the fastener.

Example 14: A method including: inserting an implantable medical lead of a medical device system through an incision site on a body of a patient; guiding an anchoring sleeve of the medical device system to the incision site, wherein the anchoring sleeve surrounds a portion of the lead; and engaging the anchoring sleeve with patient tissue at the incision site via an engagement mechanism of the anchoring sleeve, wherein the engagement mechanism is configured to assist closure of an incision in the patient tissue at the incision site.

Example 15: The method of example 14, wherein the engagement mechanism includes a plurality of tines configured to transition between a constrained state and an unconstrained state and one or more tine assemblies including: one or more surface features configured to allow a tool to grip the tine assembly; a compression fitting including one or more surface features configured to allow a tool to grip the compression fitting; and two or more tines of the plurality of tines, wherein the two or more tines extend in the direction of a longitudinal axis of the tine assembly when the two or more tines are in the constrained state, wherein the two or more tines are configured to transition between the constrained state and the unconstrained state in response to the compression fitting sliding along the longitudinal axis of the tine assembly, and wherein engaging the anchoring sleeve with the patient tissue includes sliding the compression fitting along the longitudinal axis defined by the two or more tines of the plurality of tines.

Example 16: The method of any of examples 14-15, wherein the patient tissue at the incision site includes an outer side and an inner side, and wherein the method further includes engaging the anchoring sleeve with the patient tissue on the inner side of the patient tissue at the incision site.

Example 17: The method of any of examples 14-16, wherein the engagement mechanism includes a tine coil, and wherein engaging the anchoring sleeve with the patient tissue includes rotating the anchoring sleeve to allow the tine coil to engage with patient tissue at the incision site.

Example 18: The method of any of examples 14-17, wherein the medical device system further includes an introducer configured to deliver the anchoring sleeve along the lead to the incision site, and wherein guiding the anchoring sleeve to the incision site includes guiding the introducer along the lead to the incision site.

Example 19: The method of any of examples 14-18, wherein the anchoring sleeve includes an expanding feature including: a distal end; a proximal end; a length between the distal end and the proximal end, wherein the length defines a longitudinal axis; and an engagement mechanism on an outer surface of the expanding feature configured to engage with patient tissue, wherein: the expanding feature is configured to expand in response to a compressive force along the longitudinal axis, and the expanding feature is configured to contract when not subject to the compressive force; and the method further includes: attaching the distal end of the anchoring sleeve to the implantable medical lead via a friction mechanism of the anchoring sleeve; applying a compressive force along the longitudinal axis of the expanding feature to expand the expanding feature and cause the engagement mechanism to engage with patient tissue; removing the compressive force to cause the expanding feature to contract, wherein the expanding feature assist closure of the incision in the patient tissue at the incision site when contracting.

Example 20: The method of any of examples 14-19, wherein the engagement mechanism includes: a plurality of struts, wherein the plurality of struts defines an inner diameter and an outer diameter, wherein the plurality of struts are configurable between an expanded state and a contracted state, and wherein the outer diameter defined by the plurality of struts in the expanded state is larger than the outer diameter defined by the plurality of struts in the contracted state; one or more rotatable connections between two or more struts of the plurality of struts; and a plurality of tines attached to and extending from the struts of the plurality of struts defining the outer diameter, wherein the method further includes: guiding the anchoring sleeve to the incision site when the plurality of struts are in the expanded state; rotating the anchoring sleeve to cause the plurality of tines to engage with patient tissue at the incision site; and contracting the plurality of struts, wherein contracting the plurality of struts assists closure of the incision in the patient tissue at the incision site.

Example 21: An anchoring sleeve including an engagement mechanism configurable between an expanded state and a contracted state, wherein: the engagement mechanism is configured to engage with patient tissue at an incision site in patient tissue, and the engagement mechanism is configured to assist closure of an incision in the patient tissue at the incision site when the engagement mechanism transitions from the expanded state to the contracted state.

Various aspects of the disclosure have been described. These and other aspects are within the scope of the following claims.

## Claims

1. A medical device system comprising:
an implantable medical lead (102) configured to be delivered through an incision site (108) on a body of a patient (104); and
an anchoring sleeve (106) surrounding a portion of the lead and comprising an engagement mechanism (316) configured to:
engage with patient tissue at the incision site; and
assist closure of an incision in the patient tissue at the incision site.

2. The medical device system of claim 1, wherein the engagement mechanism comprises a plurality of tines (532) configured to transition between a constrained state and an unconstrained state, and wherein transitioning from the constrained state to the unconstrained state causes the plurality of tines to engage with the patient tissue and assist closure of the incision in the patient tissue at the incision site.

3. The medical device system of claim 2, further comprising one or more tine assemblies (530) comprising:
one or more surface features (536, 538) configured to allow a tool to grip the tine assembly;
a compression fitting (534) comprising one or more surface features configured to allow a tool to grip the compression fitting; and
two or more tines of the plurality of tines, wherein:
the two or more tines extend in the direction of a longitudinal axis of the tine assembly when the two or more tines are in the constrained state; and
the two or more tines are configured to transition between the constrained state and the unconstrained state in response to the compression fitting sliding along the longitudinal axis of the tine assembly.

4. The medical device system of claims 1-3, wherein the patient tissue at the incision site comprises an outer side and an inner side, and wherein the engagement mechanism is configured to engage with the distal side of the patient tissue at the incision site.

5. The medical device system of claims 1-4, further comprising an introducer (420) configured to deliver the anchoring sleeve along the lead to the incision site.

6. The medical device system of claims 1-5, wherein the anchoring sleeve is configurable between an expanded state and a contracted state, wherein transitioning from the expanded state to the contracted state assists closure of the incision in the patient tissue at the incision site.

7. The medical device system of claims 1-6, further comprising an introducer (420) configured to deliver the anchoring sleeve along the lead to the incision site, wherein:
the introducer comprises:
a first inner diameter defining a first lumen, wherein the lead is sized to be inserted through the first lumen; and
a first outer diameter comprising a low-friction material; and
the anchoring sleeve comprises a second inner diameter defining a second lumen, wherein the first outer diameter of the introducer is sized to be inserted through the second lumen.

8. The medical device system of claim 7, wherein the anchoring sleeve is configurable between an expanded and a contracted state, and wherein the introducer holds the anchoring sleeve in the expanded state when the introducer is inserted through the second lumen.

9. The medical device system of claims 1-8, wherein the anchoring sleeve comprises an expanding feature (742) configurable between an expanded and a contracted state, wherein transitioning from the expanded state to the contracted state causes the expanding feature to assist closure of the incision in the patient tissue at the incision site.

10. The medical device system of claim 9, wherein expanding feature comprises:
a distal end (748);
a proximal end; and
a length between the distal end and the proximal end, wherein the length defines a longitudinal axis,
wherein the expanding feature is configured to expand in response to a compressive force along the longitudinal axis, and the expanding feature is configured to contract when not subject to the compressive force.

11. The medical device system of claim 10, wherein the expanding feature further comprises:
a friction mechanism (744) near the distal end wherein the friction mechanism is configured to attach the anchoring sleeve to a portion of the lead;
an envelope (750); and
one or more spines (752) in contact with the envelope, wherein the one or more spines are configured to expand the envelope when the spines are subject to the compressive force along the longitudinal axis of the expanding feature, and wherein the one or more spines are configured to contract the envelope when not subject to the compressive force.

12. The medical device system of claims 1-11, wherein the engagement mechanism comprises:
a plurality of struts, wherein the plurality of struts defines an inner diameter and an outer diameter, wherein the struts are configurable between an expanded state and a contracted state, and wherein the outer diameter defined by the plurality of struts in the expanded state is larger than the outer diameter defined by the plurality of struts in the contracted state;
one or more rotatable connections between two or more struts of the plurality of struts; and
a plurality of tines attached to and extending from the struts of the plurality of struts defining the outer diameter, wherein the plurality of tines is configured to engage with the patient tissue at the incision site, and wherein to assist closure of the incision in the patient tissue at the incision site, the engagement mechanism is configured to at least partially close the incision when the plurality of struts transitions from the expanded state to the contracted state.

13. The medical device system of claims 1-12, wherein the engagement mechanism comprises:
a plurality of struts, wherein each strut of the plurality of struts rotatably connects to at least one other strut of the plurality of struts, and wherein the plurality of struts defines a parallelogram; and
a fastener rigidly attached to a first side of the parallelogram defined by the plurality of struts and slidably attached to a second side of the parallelogram defined by the plurality of struts, wherein the plurality of struts is configured to close in response to a pulling force applied to the fastener.

## Patentansprüche

1. System der medizinischen Vorrichtung, umfassend:
eine implantierbare medizinische Leitung (102), die konfiguriert ist, um durch eine Inzisionsstelle (108) an einem Körper eines Patienten (104) abgegeben zu werden; und
eine Verankerungshülse (106), die einen Abschnitt der Leitung umgibt und umfassend einen Eingriffsmechanismus (316), der konfiguriert ist zum:
Ineingriffnehmen von Patientengewebe an der Inzisionsstelle; und
Unterstützen eines Verschlusses einer Inzision in dem Patientengewebe an der Inzisionsstelle.

2. System der medizinischen Vorrichtung nach Anspruch 1, wobei der Eingriffsmechanismus eine Vielzahl von Zinken (532) umfasst, die konfiguriert sind, um zwischen einem eingeschränkten Zustand und einem uneingeschränkten Zustand überzugehen, und wobei das Übergehen von dem eingeschränkten Zustand in den uneingeschränkten Zustand bewirkt, dass die Vielzahl von Zinken das Patientengewebe in Eingriff nimmt und den Verschluss der Inzision in dem Patientengewebe an der Inzisionsstelle unterstützt.

3. System der medizinischen Vorrichtung nach Anspruch 2, ferner umfassend eine oder mehrere Zinkenanordnungen (530), umfassend:
ein oder mehrere Oberflächenmerkmale (536, 538), die konfiguriert sind, um einem Werkzeug zu ermöglichen, die Zinkenanordnung zu greifen;
eine Klemmverschraubung (534), umfassend ein oder mehrere Oberflächenmerkmale, die konfiguriert sind, um einem Werkzeug zu ermöglichen, die Klemmverschraubung zu greifen; und
zwei oder mehr Zinken der Vielzahl von Zinken, wobei:
sich die zwei oder mehr Zinken in der Richtung einer Längsachse der Zinkenanordnung erstrecken, wenn sich die zwei oder mehr Zinken in dem eingeschränkten Zustand befinden; und
die zwei oder mehr Zinken konfiguriert sind, um zwischen dem eingeschränkten Zustand und dem uneingeschränkten Zustand als Reaktion auf ein Gleiten des Klemmverschraubung entlang der Längsachse der Zinkenanordnung überzugehen.

4. System der medizinischen Vorrichtung nach den Ansprüchen 1 bis 3, wobei das Patientengewebe an der Inzisionsstelle eine Außenseite und eine Innenseite umfasst, und wobei der Eingriffsmechanismus konfiguriert ist, um die distale Seite des Patientengewebes an der Inzisionsstelle in Eingriff zu nehmen.

5. System der medizinischen Vorrichtung nach den Ansprüchen 1 bis 4, ferner umfassend eine Einführeinrichtung (420), die konfiguriert ist, um die Verankerungshülse entlang der Leitung zu der Inzisionsstelle abzugeben.

6. System der medizinischen Vorrichtung nach den Ansprüchen 1 bis 5, wobei die Verankerungshülse zwischen einem expandierten Zustand und einem kontrahierten Zustand konfigurierbar ist, wobei das Übergehen von dem expandierten Zustand in den kontrahierten Zustand den Verschluss der Inzision in dem Patientengewebe an der Inzisionsstelle unterstützt.

7. System der medizinischen Vorrichtung nach den Ansprüchen 1 bis 6, ferner umfassend eine Einführeinrichtung (420), die konfiguriert ist, um die Verankerungshülse entlang der Leitung zu der Inzisionsstelle abzugeben, wobei:
die Einführeinrichtung umfasst:
einen ersten Innendurchmesser, der ein erstes Lumen definiert, wobei die Leitung bemessen ist, um durch das erste Lumen eingeführt zu werden; und
einen ersten Außendurchmesser, umfassend ein reibungsarmes Material; und
die Verankerungshülse einen zweiten Innendurchmesse umfasst, der ein zweites Lumen definiert, wobei der erste Außendurchmesser der Einführeinrichtung bemessen ist, um durch das zweite Lumen eingeführt zu werden.

8. System der medizinischen Vorrichtung nach Anspruch 7, wobei die Verankerungshülse zwischen einem expandierten und einem kontrahierten Zustand konfigurierbar ist, und wobei die Einführeinrichtung die Verankerungshülse in dem expandierten Zustand hält, wenn die Einführeinrichtung durch das zweite Lumen eingeführt wird.

9. System der medizinischen Vorrichtung nach den Ansprüchen 1 bis 8, wobei die Verankerungshülse ein expandierendes Merkmal (742) umfasst, das zwischen einem expandierten und einem kontrahierten Zustand konfigurierbar ist, wobei das Übergehen von dem expandierten Zustand in den kontrahierten Zustand bewirkt, dass das expandierende Merkmal den Verschluss der Inzision in dem Patientengewebe an der Inzisionsstelle unterstützt.

10. System der medizinischen Vorrichtungen nach Anspruch 9, wobei das expandierende Merkmal umfasst:
eine distale Spitze (748);
ein proximales Ende; und
eine Länge zwischen dem distalen Ende und dem proximalen Ende, wobei die Länge eine Längsachse definiert,
wobei das expandierende Merkmal konfiguriert ist, um als Reaktion auf eine Druckkraft entlang der Längsachse zu expandieren, und das expandierende Merkmal konfiguriert ist, um zu kontrahieren, wenn es der Druckkraft nicht ausgesetzt ist.

11. System der medizinischen Vorrichtung nach Anspruch 10, wobei das expandierende Merkmal ferner umfasst:
einen Reibungsmechanismus (744) nahe dem distalen Ende, wobei der Reibungsmechanismus konfiguriert ist, um die Verankerungshülse an einem Abschnitt der Leitung zu befestigen;
eine Hülle (750); und
einen oder mehrere Grate (752) in Kontakt mit der Hülle, wobei der eine oder die mehreren Grate konfiguriert sind, um die Hülle zu expandieren, wenn der Grat der Druckkraft entlang der Längsachse des expandierenden Merkmals ausgesetzt sind, und wobei der eine oder die mehreren Grate konfiguriert sind, um die Hülle zu kontrahieren, wenn sie der Druckkraft nicht ausgesetzt sind.

12. System der medizinischen Vorrichtung nach den Ansprüchen 1 bis 11, wobei der Eingriffsmechanismus umfasst:
eine Vielzahl von Streben, wobei die Vielzahl von Streben einen Innendurchmesser und einen Außendurchmesser definiert, wobei die Streben zwischen einem expandierten Zustand und einem kontrahierten Zustand konfigurierbar sind, und wobei der Außendurchmesser, der durch die Vielzahl von Streben in dem expandierten Zustand definiert wird, größer als der Außendurchmesser ist, der durch die Vielzahl von Streben in dem kontrahierten Zustand definiert wird;
eine oder mehrere drehbare Verbindungen zwischen zwei oder mehr Streben der Vielzahl von Streben; und
eine Vielzahl von Zinken, die an den Streben der Vielzahl von Streben, die den Außendurchmesser definieren, befestigt sind und sich von diesen erstrecken, wobei die Vielzahl von Zinken konfiguriert ist, um das Patientengewebe an der Inzisionsstelle in Eingriff zu nehmen, und wobei, um den Verschluss der Inzision in dem Patientengewebe an der Inzisionsstelle zu unterstützen, der Eingriffsmechanismus konfiguriert ist, um die Inzision mindestens teilweise zu schließen, wenn die Vielzahl von Streben von dem expandierten Zustand in den kontrahierten Zustand übergeht.

13. System der medizinischen Vorrichtung nach den Ansprüchen 1 bis 12, wobei der Eingriffsmechanismus umfasst:
eine Vielzahl von Streben, wobei jede Strebe der Vielzahl von Streben mit mindestens einer anderen Strebe der Vielzahl von Streben drehbar verbunden ist, und wobei die Vielzahl von Streben ein Parallelogramm definiert; und
ein Befestigungselement, das an einer ersten Seite des Parallelogramms, das durch die Vielzahl von Streben definiert ist, starr befestigt ist und an einer zweiten Seite des Parallelogramms, das durch die Vielzahl von Streben definiert ist, verschiebbar befestigt ist, wobei die Vielzahl von Streben konfiguriert ist, sich als Reaktion auf eine Zugkraft zu schließen, die auf das Befestigungselement ausgeübt wird.

## Revendications

1. Système de dispositif médical comprenant :
une dérivation médicale implantable (102) conçue pour être délivrée à travers un site d'incision (108) sur un corps d'un patient (104) ; et
un manchon d'ancrage (106) entourant une partie de la dérivation et comprenant un mécanisme de mise en prise (316) conçu pour :
venir en prise avec un tissu de patient au niveau du site d'incision ; et
aider à la fermeture d'une incision dans le tissu de patient au niveau du site d'incision.

2. Système de dispositif médical selon la revendication 1, dans lequel le mécanisme de mise en prise comprend une pluralité de dents (532) conçues pour effectuer une transition entre un état contraint et un état non contraint, et dans lequel la transition de l'état contraint à l'état non contraint amène la pluralité de dents à venir en prise avec le tissu de patient et à aider à la fermeture de l'incision dans le tissu de patient au niveau du site d'incision.

3. Système de dispositif médical selon la revendication 2, comprenant en outre un ou plusieurs ensembles de dents (530) comprenant :
une ou plusieurs caractéristiques de surface (536, 538) conçues pour permettre à un outil de saisir l'ensemble de dents ;
un raccord à compression (534) comprenant une ou plusieurs caractéristiques de surface conçues pour permettre à un outil de saisir le raccord à compression ; et
deux dents ou plus de la pluralité de dents, dans lequel :
les deux dents ou plus s'étendent dans la direction d'un axe longitudinal de l'ensemble de dents lorsque les deux dents ou plus sont dans l'état contraint ; et
les deux dents ou plus sont conçues pour effectuer une transition entre l'état contraint et l'état non contraint en réponse au raccord à compression coulissant le long de l'axe longitudinal de l'ensemble de dents.

4. Système de dispositif médical selon les revendications 1 à 3, dans lequel le tissu de patient au niveau du site d'incision comprend un côté externe et un côté interne, et dans lequel le mécanisme de mise en prise est conçu pour venir en prise avec le côté distal du tissu de patient au niveau du site d'incision.

5. Système de dispositif médical selon les revendications 1 à 4, comprenant en outre un introducteur (420) conçu pour délivrer le manchon d'ancrage le long de la dérivation vers le site d'incision.

6. Système de dispositif médical selon les revendications 1 à 5, dans lequel le manchon d'ancrage est configurable entre un état expansé et un état contracté, dans lequel la transition de l'état expansé à l'état contracté aide à la fermeture de l'incision dans le tissu de patient au niveau du site d'incision.

7. Système de dispositif médical selon les revendications 1 à 6, comprenant en outre un introducteur (420) conçu pour délivrer le manchon d'ancrage le long de la dérivation vers le site d'incision, dans lequel :
l'introducteur comprend :
un premier diamètre interne définissant une première lumière, dans lequel la dérivation est dimensionnée pour être insérée à travers la première lumière ; et
un premier diamètre externe comprenant un matériau à faible frottement ; et
le manchon d'ancrage comprend un second diamètre interne définissant une seconde lumière, dans lequel le premier diamètre externe de l'introducteur est dimensionné pour être inséré à travers la seconde lumière.

8. Système de dispositif médical selon la revendication 7, dans lequel le manchon d'ancrage est configurable entre un état expansé et un état contracté, et dans lequel l'introducteur maintient le manchon d'ancrage dans l'état expansé lorsque l'introducteur est inséré à travers la seconde lumière.

9. Système de dispositif médical selon les revendications 1 à 8, dans lequel le manchon d'ancrage comprend une caractéristique d'expansion (742) configurable entre un état expansé et un état contracté, dans lequel la transition de l'état expansé à l'état contracté amène la caractéristique d'expansion à aider à la fermeture de l'incision dans le tissu de patient au niveau du site d'incision.

10. Système de dispositif médical selon la revendication 9, dans lequel la caractéristique d'expansion comprend :
une extrémité distale (748) ;
une extrémité proximale ; et
une longueur entre l'extrémité distale et l'extrémité proximale, dans lequel la longueur définit un axe longitudinal,
dans lequel la caractéristique d'expansion est conçue pour s'expanser en réponse à une force de compression le long de l'axe longitudinal, et la caractéristique d'expansion est conçue pour se contracter lorsqu'elle n'est pas soumise à la force de compression.

11. Système de dispositif médical selon la revendication 10, dans lequel la caractéristique d'expansion comprend en outre :
un mécanisme de frottement (744) près de l'extrémité distale dans lequel le mécanisme de frottement est conçu pour attacher le manchon d'ancrage à une partie de la dérivation ;
une enveloppe (750) ; et
un ou plusieurs éléments d'armature (752) en contact avec l'enveloppe, dans lequel le ou les éléments d'armature sont conçus pour expanser l'enveloppe lorsque les éléments d'armature sont soumis à la force de compression le long de l'axe longitudinal de la caractéristique d'expansion, et dans lequel le ou les éléments d'armature sont conçus pour contracter l'enveloppe lorsqu'ils ne sont pas soumis à la force de compression.

12. Système de dispositif médical selon les revendications 1 à 11, dans lequel le mécanisme de mise en prise comprend :
une pluralité d'entretoises, dans lequel la pluralité d'entretoises définit un diamètre interne et un diamètre externe, dans lequel les entretoises sont configurables entre un état expansé et un état contracté, et dans lequel le diamètre externe défini par la pluralité d'entretoises dans l'état expansé est plus grand que le diamètre externe défini par la pluralité d'entretoises dans l'état contracté ;
une ou plusieurs liaisons rotatives entre deux entretoises ou plus de la pluralité d'entretoises ; et
une pluralité de dents attachées aux, et s'étendant à partir des entretoises de la pluralité d'entretoises définissant le diamètre externe, dans lequel la pluralité de dents est conçue pour venir en prise avec le tissu de patient au niveau du site d'incision, et dans lequel pour aider à la fermeture de l'incision dans le tissu de patient au niveau du site d'incision, le mécanisme de mise en prise est conçu pour fermer au moins partiellement l'incision lorsque la pluralité d'entretoises effectue une transition de l'état expansé à l'état contracté.

13. Système de dispositif médical selon les revendications 1 à 12, dans lequel le mécanisme de mise en prise comprend :
une pluralité d'entretoises, dans lequel chaque entretoise de la pluralité d'entretoises se relie de manière rotative à au moins une autre entretoise de la pluralité d'entretoises, et dans lequel la pluralité d'entretoises définit un parallélogramme ; et
un élément de fixation fixé de manière solidaire à un premier côté du parallélogramme défini par la pluralité d'entretoises et fixé de manière coulissante à un second côté du parallélogramme défini par la pluralité d'entretoises, dans lequel la pluralité d'entretoises est conçue pour se fermer en réponse à une force de traction appliquée à l'élément de fixation.
